# EUROPEAN PATENT SPECIFICATION

(11) **EP 4 431 016 B1**
(45) Date of publication and mention of the grant of the patent: **04.11.2026**
(21) Application number: 23753042.3
(22) Date of filing: 30.01.2023
(51) Int. Cl.: A61B 5/11, A61B 5/00, A61B 5/107, G16H 20/30

(54) **ELECTRONIC DEVICE AND WEARABLE DEVICE FOR PROVIDING PHYSICAL ABILITY MEASUREMENT MODE, AND OPERATION METHODS THEREOF**
ELEKTRONISCHE VORRICHTUNG UND WEARABLE-VORRICHTUNG ZUR BEREITSTELLUNG EINES MESSMODUS FÜR PHYSIKALISCHE FÄHIGKEITEN UND BETRIEBSVERFAHREN DAFÜR
DISPOSITIF ÉLECTRONIQUE ET DISPOSITIF PORTABLE POUR FOURNIR UN MODE DE MESURE DE CAPACITÉ PHYSIQUE, ET PROCÉDÉS DE FONCTIONNEMENT ASSOCIÉS

(30) Priority: 14.02.2022 KR 20220019057; 16.09.2022 KR 20220117341
(43) Date of publication of application: 18.09.2024
(73) Proprietor: Samsung Electronics Co., Ltd., Suwon-si, Gyeonggi-do 16677 (KR)
(72) Inventor: KIM, Kyungrock, Suwon-si Gyeonggi-do 16677 (KR); LEE, Jusuk, Suwon-si Gyeonggi-do 16677 (KR); PARK, Jiyoung, Suwon-si Gyeonggi-do 16677 (KR); SEO, Keehong, Suwon-si Gyeonggi-do 16677 (KR); LEE, Hwangjae, Suwon-si Gyeonggi-do 16677 (KR); LIM, Bokman, Suwon-si Gyeonggi-do 16677 (KR)
(74) Representative: Gulde & Partner
(86) International application number: PCT/KR2023/001329
(87) International publication number: WO 2023/153691

(56) References cited:
- WO-A1-2019/175899
- JP-A- 2021 003 605
- KR-A- 20160 090 088
- KR-A- 20160 090 088
- KR-A- 20170 013 060
- KR-A- 20170 013 060
- KR-A- 20190 008 519
- KR-A- 20190 008 519
- US-A1- 2014 142 475
- US-A1- 2016 095 538
- US-A1- 2021 259 579
- US-A1- 2021 259 579
- US-A1- 2021 321 906
- NISWANDER WESLEY ET AL: "Evaluating the Impact of IMU Sensor Location and Walking Task on Accuracy of Gait Event Detection Algorithms", SENSORS, vol. 21, no. 12, 9 June 2021 (2021-06-09), CH, pages 3989, XP093285633, ISSN: 1424-8220, DOI: 10.3390/s21123989
- MARANESI E ET AL: "Assessment of walking speed by a goniometer-based method", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 1202 - 1205, XP032675689, [retrieved on 20141102], DOI: 10.1109/EMBC.2014.6943812

## Description

### BACKGROUND

### 1. Field

Certain example embodiments relate to an electronic device and a wearable device for providing a physical ability measurement mode, a method of operating the electronic device, and a method of operating the wearable device.

### 2. Description of Related Art

Various measurement methods may be used to evaluate a person's gait health, muscle strength, fall risk, and the like. Among such measurement methods, measurement methods widely used in the field of rehabilitation include, for example, a 10-meter walk test (MWT), a 6-minute walk test (mWT), a short physical performance battery (SPPB) test, a timed up and go (TUG) test, and the like. If a person for a measurement and a simple tool (e.g., a stopwatch, a chair, a tape measure, etc.) are provided, measurement is possible in the measurement methods.

KR 2016 0090088 A discloses a walking assistance device that uses pressure data from the user's sole to estimate their walking motion. The device includes a vibrator that provides feedback to the user.

US 2021/321906 A1 discloses a system for evaluating a person's body condition based on their walking patterns. It uses a measurement apparatus to continuously monitor the walking and an arithmetic operation apparatus to analyze the data. Periods of "stable walking" are extracted to minimize the impact of external factors on the analysis. The system then evaluates the walking speed and other metrics during these stable walking periods.

US 2021/259579 A1 discloses a device for treating Idiopathic Toe Walking (ITW) in children. The device includes pressure sensors on the insole of a shoe to detect the child's gait pattern. If the child exhibits a toe-to-toe gait, the device can provide haptic feedback, encouraging them to adopt a heel-to-toe pattern.

KR 2017 0013060 A discloses a walking assistance apparatus that uses sensors to detect the user's landing time and joint positions to provide assistance. The device has a controller that initiates and terminates the output of an assist torque profile to the user's legs, helping them walk.

KR 2019 0008519 A discloses a gait profiler system which involves a system with sensors to gather information about a user's gait. The system uses this information to calculate a gait profile, which is then optimized based on the gathered data.

US 2016/095538 A1 discloses a method and apparatus for recognizing gait motion using a neural network. The system utilizes hip joint angle information and acceleration data to identify different gait patterns, such as walking up or down stairs, walking on a slope, and level walking. The recognized gait motion is then transmitted to a walking assistance apparatus, enabling it to provide appropriate assistance to the user based on their current movement.

US 2014/142475 A1 discloses a movement assistance device, and specifically a powered orthosis designed for individuals with paraplegia. The device features a distributed embedded system that controls the motors in the orthosis to provide assistance based on sensor data and user input.

WO 2019/175899 A1 discloses a wearable device for gait analysis, consisting of a pair of shoes/socks and multiple embedded wearable braces (ankle, calf, thigh, and hip). The device integrates motion sensors, pressure sensors, electromyography (EMG) sensors, and distance measurement sensors to analyze lower limb kinematics and kinetics. It measures parameters like stride length, plantar pressure, joint angles, and muscle activity. The system includes a wireless module for data transmission, a gait synchronization unit, and onboard power management.

### SUMMARY

The invention is defined in the appended set of claims.

### BRIEF DESCRIPTION OF THE DRAWINGS

The above and other aspects, features, and advantages of certain embodiments of the present disclosure will be more apparent from the following detailed description, taken in conjunction with the accompanying drawings, in which:
FIG. 1 illustrates an overview of a wearable device worn on a body of a user according to an example embodiment;
FIG. 2 is a diagram illustrating a management system including a wearable device and an electronic device according to an example embodiment;
FIG. 3 is a diagram illustrating a front surface of a wearable device according to an example embodiment;
FIG. 4 is a diagram illustrating a side of a wearable device according to an example embodiment;
FIGS. 5A and 5B are diagrams illustrating a configuration of a control system of a wearable device according to an example embodiment;
FIG. 6 is a diagram illustrating an interaction between a wearable device and an electronic device according to an example embodiment;
FIG. 7 is a diagram illustrating a configuration of an electronic device according to an example embodiment;
FIG. 8 is a diagram illustrating an operating method of an electronic device and a wearable device to measure a physical ability of a user according to an example embodiment;
FIG. 9 is a diagram illustrating a user interface (UI) provided to a user through an electronic device to measure a walking ability, according to an example embodiment;
FIG. 10 is a diagram illustrating an example of measuring a walking ability using a wearable device and an electronic device according to an example embodiment;
FIG. 11 is a flowchart illustrating a method of measuring a walking ability of a user according to an example embodiment;
FIG. 12 is a diagram illustrating an example of extracting feature points from sensor data for a measurement of a walking ability according to an example embodiment;
FIG. 13 is a diagram illustrating an example of estimating a spatial gait index of a user according to an example embodiment;
FIG. 14 is a flowchart illustrating a method of measuring a physical ability through a timed up and go (TUG) test according to an example embodiment;
FIG. 15 is a diagram illustrating an example of extracting a feature point of sensor data to measure a physical ability in a TUG test according to an example embodiment;
FIG. 16 is a flowchart illustrating a method of measuring a physical ability through a five times sit to stand (5×STS) test according to an example embodiment;
FIG. 17 is a diagram illustrating an example of extracting a feature point of sensor data to measure a physical ability in a 5×STS test according to an example embodiment; and
FIG. 18 is a flowchart illustrating a method of measuring a physical ability through a short physical performance battery (SPPB) test according to an example embodiment.

### DETAILED DESCRIPTION

The following detailed structural or functional description is provided as an example only and various alterations and modifications may be made to embodiments. Accordingly, the embodiments are not construed as limited to the disclosure and should be understood to include all changes, equivalents, and replacements within the idea and the technical scope of the disclosure.

As used herein, the singular forms "a", "an", and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, the embodiments will be described in detail with reference to the accompanying drawings. When describing the embodiments with reference to the accompanying drawings, like reference numerals refer to like elements and a repeated description related thereto will be omitted.

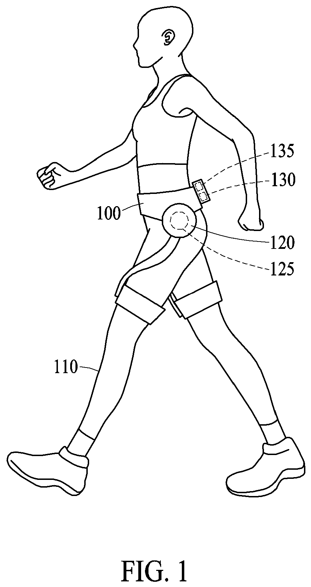

FIG. 1 illustrates an overview of a wearable device worn on a body of a user according to an embodiment.

Referring to FIG. 1, in an embodiment, a wearable device 100 may be a device worn on a body of a user 110 to assist the user 110 in walking, exercising, and/or working. In an embodiment, the wearable device 100 may also be used to measure a physical ability (e.g., a walking ability, and an exercise ability) of the user 110. In embodiments, the term "wearable device" may be a "wearable robot," "walking assistance device," or "exercise assistance device". The user 110 may be a human or an animal, but is not limited thereto. The wearable device 100 may be worn on a body (e.g., a lower body, e.g., a leg, an ankle, a knee, etc., an upper body, e.g., a trunk, an arm, a wrist, etc., or a waist) of the user 110 to apply an assistance force and/or an external force of a resistance force to a motion of the body of the user 110. The assistance force may be a force applied in the same direction as a direction of a body motion of the user 110 and may represent a force to assist the body motion of the user 110. The resistance force may be a force applied in a direction opposite to the direction of the body motion of the user 110 and may represent a force to hinder the body motion of the user 110. The term "resistance force" may also be referred to as an "exercise load."

In an embodiment, when the wearable device 100 operates in a walking assistance mode for assisting walking of the user 110, the wearable device 100 may assist walking of the user 110 by applying an assistance force generated from a driving module 120 of the wearable device 100 to the body of the user 110. The wearable device 100 may allow the user 110 to walk independently or to walk for a long time by providing a force required for walking of the user 110, to expand a walking ability of the user 110. The wearable device 100 may assist a user in improving an abnormal walking habit or abnormal walking posture of the user.

In an embodiment, when the wearable device 100 operates in an exercise assistance mode for enhancing an exercise effect of the user 110, the wearable device 100 may hinder a body motion of the user 110 or provide a resistance to the body motion of the user 110, by applying a resistance force generated from the driving module 120 to the body of the user 110. When the wearable device 100 is a hip-type wearable device that is wearable on a waist (or a pelvis) and a leg (e.g., a thigh) of the user 110, the wearable device 100 may provide an exercise load to the body motion of the user 110 while being worn on the leg, to further strengthen an exercise effect on the leg of the user 110. In an embodiment, the wearable device 100 may also apply an assistance force to the body of the user 110 to assist the body motion of the user 110 in the exercise assistance mode. In an embodiment, the wearable device 100 may combine the assistance force and resistance force for each exercise interval or time interval, and provide a combination of the assistance force and resistance force, in a similar manner to providing an assistance force in some exercise intervals and providing a resistance force in some exercise intervals in the exercise assistance mode.

In an embodiment, when the wearable device 100 operates in a physical ability measurement mode for measuring the physical ability of the user 110, the wearable device 100 measures motion information of a user, using sensors (an angle sensor 125, and an inertial measurement unit (IMU) 135) included in the wearable device 100 while the user is walking or (not according to the claimed invention) exercising, and may evaluate a physical ability of the user based on the measured motion information.

In embodiments of the present disclosure, for convenience of description, the wearable device 100 is described as an example of a hip-type wearable device, as illustrated in FIG. 1, but the embodiments are not limited thereto. As described above, the wearable device 100 may also be worn on body parts (not according to the claimed invention: e.g., an upper arm, a lower arm, a hand, a calf, and a foot) other than the waist and the leg (e.g., a thigh). A shape and a configuration of the wearable device 100 may vary depending on a body part on which the wearable device 100 is to be worn.

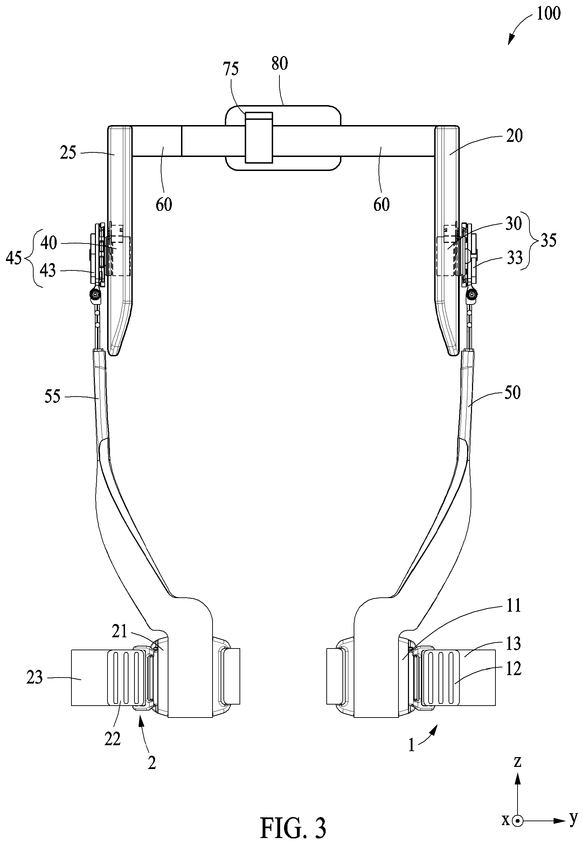

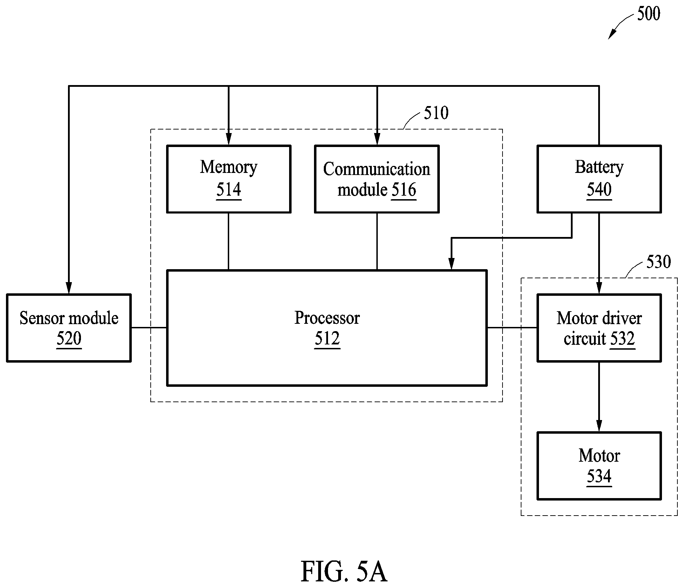

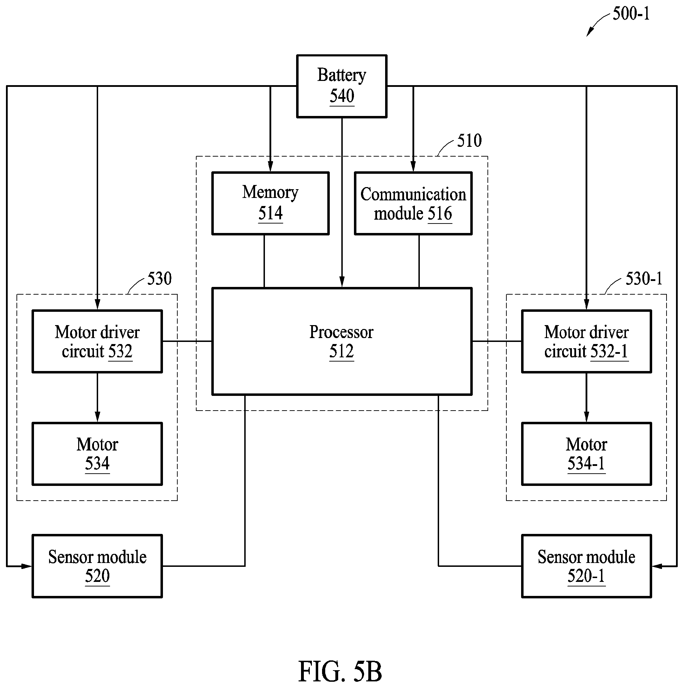

According to an embodiment, the wearable device 100 may include a support frame (e.g., leg support frames 50 and 55 and waist support frames 20 and 25 of FIG. 3) configured to support the body of the user 110 when the wearable device 100 is worn on the body of the user 110, a sensor module (e.g., a sensor module 520 of FIG. 5A) configured to obtain sensor data including motion information on a body motion (e.g., a motion of a leg, and a motion of an upper body) of a user, the driving module 120 (e.g., driving modules 35 and 45 of FIG. 3) configured to generate an external force to be applied to a leg of a user, and a control module 130 (e.g., a control module 510 of FIGS. 5A and 5B) configured to control the wearable device 100.

The sensor module may include the angle sensor 125 and/or the IMU 135. The angle sensor 125 may measure a hip joint angle value of the user 110. The angle sensor 125 may include, for example, an encoder and/or a hall sensor. In an embodiment, angle sensor(s) 125 may be disposed near a left hip joint and a right hip joint, respectively, and may measure a hip joint angle value of a left hip joint of the user 110 and a hip joint angle value of a right hip joint of the user 110. The hip joint angle value of the left hip joint may correspond to an angle of a left leg of the user 110, and the hip joint angle value of the right hip joint may correspond to an angle of a right leg of the user 110. The IMU 135 (or an inertial sensor) may measure a change in an acceleration and a rotation speed according to a motion of the user 110. For example, the IMU 135 may measure an upper body motion value of the user 110. The IMU 135 may include an acceleration sensor, and/or an angular velocity sensor.

In an embodiment, the control module 130 and the IMU 135 may be arranged in a housing (e.g., a housing 80 of FIG. 3) of the wearable device 100. The housing may be formed or attached to the outside of the support frame of the wearable device 100, and may be disposed behind the waist when the user 110 wears the wearable device 100, however, the embodiments are not limited thereto.

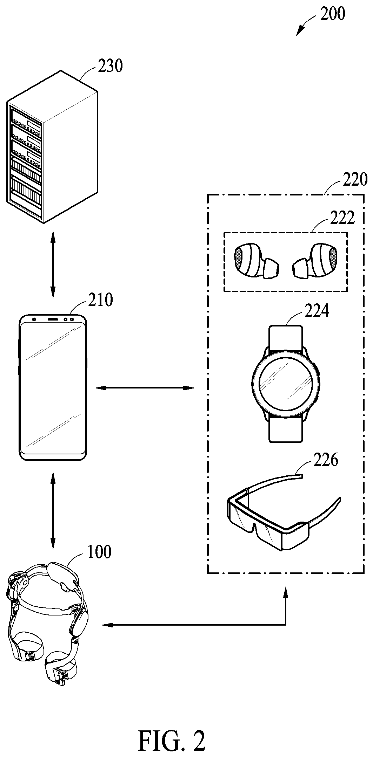

The wearable device 100 provides a physical ability measurement (or evaluation) function of measuring (or evaluating) the physical ability of the user 110 by interworking with an electronic device (e.g., an electronic device 210 of FIG. 2). When the user 110 wearing the wearable device 100 performs operations according to a guide provided through a program (e.g., an application) executed in the electronic device, the wearable device 100 may obtain sensor data according to a motion of a user, using the sensor module. The electronic device may analyze the sensor data obtained by the wearable device 100, estimate a physical ability (e.g., a walking ability, a muscle strength, a potential fall, etc.) of the user, calculate (e.g., determine) physical ability information on the estimated physical ability, and provide the user 110 with the physical ability information. The electronic device and the wearable device 100 may provide various measurement schemes (e.g., a walking ability test, a timed up and go (TUG) test, a five times sit to stand (5×STS) test, and a short physical performance battery (SPPB) test) to measure a physical ability of a user. As a measurement scheme that may be provided, a new measurement scheme may be added, or a detailed process of an existing measurement scheme may be updated. The user 110 may select a measurement scheme to be performed, and a guide to perform the measurement scheme selected by the user 110 may be provided to the user 110.

The wearable device 100 and the electronic device may periodically measure a physical ability of a user and provide the user with physical ability information. In an embodiment, the physical ability information of the user may also be transmitted to a terminal of a person (e.g., a family member, a medical professional, or an exercise instructor) registered in the electronic device.

FIG. 2 is a diagram illustrating a management system including a wearable device and an electronic device according to an embodiment.

Referring to FIG. 2, a management system 200 may include a wearable device 100 to assist a body motion of a user, the electronic device 210, other wearable devices 220, and a server 230. In an embodiment, at least one (e.g., the other wearable devices 220 or the server 230) of the above devices may be omitted from the management system 200, or one or more other devices (e.g., an exclusive controller for the wearable device 100) may be added to the management system 200.

In an embodiment, the wearable device 100 may be worn on a body of the user in the walking assistance mode to assist a motion of the user. For example, the wearable device 100 may be worn on a leg of the user and generate an assistance force to assist a motion of the leg of the user, to assist the user in walking. In an embodiment, to enhance an exercise effect of the user in the exercise assistance mode, the wearable device 100 may generate a resistance force to hinder a motion of the body of the user motion and apply the resistance force to the body.

In an embodiment, the wearable device 100 is used to measure a physical ability of the user by interworking with the electronic device 210. The wearable device 100 operates in a physical ability measurement mode, which is a mode for measuring the physical ability of the user, under a control of the electronic device 210, and transmits sensor data obtained by a motion of the user in the physical ability measurement mode to electronic device 210. The electronic device 210 may estimate the physical ability of the user by analyzing the sensor data received from the wearable device 100.

The electronic device 210 may communicate with the wearable device 100, remotely control the wearable device 100, or provide state information (e.g., an amount of charge remaining in a battery) of the wearable device 100 to the user. The electronic device 210 may receive sensor data obtained by a sensor of the wearable device 100 from the wearable device 100, and may determine a physical condition or physical ability of the user based on the received sensor data. In an embodiment, the electronic device 210 may execute a program (e.g., an application) to control the wearable device 100, and the user may adjust a set value (e.g., an intensity of torque, and/or an audio volume) or an operation of the wearable device 100. According to an embodiment, the electronic device 210 may be various types of devices. The electronic device 210 may include, for example, a portable communication device (e.g., a smartphone), a computer device, a portable multimedia device, or a home appliance device, but is not limited thereto.

According to an embodiment, the electronic device 210 may be connected to the server 230 using short-range wireless communication or cellular communication. The server 230 may receive user information (e.g., name, age, gender) on a user who uses the wearable device 100 and/or physical ability information associated with a corresponding user from the electronic device 210, and may store and manage the received user information and the received physical ability information. The server 230 may provide the electronic device 210 with various exercise programs or physical ability measurement programs that may be provided to the user by the wearable device 100.

According to an embodiment, the wearable device 100 and/or the electronic device 210 may be connected, directly or indirectly, to the other wearable devices 220. The other wearable devices 220 may include, for example, wireless earphones 222, a smartwatch 224, or smart glasses 226, but are not limited thereto. In an embodiment, physical ability information generated by the electronic device 210 and/or the state information of the wearable device 100 may be transmitted to the other wearable devices 220 to be provided to the user through the other wearable devices 220. In an embodiment, the wearable device 100, the electronic device 210, and the other wearable devices 220 may be connected to each other through wireless communication (e.g., Bluetooth communication).

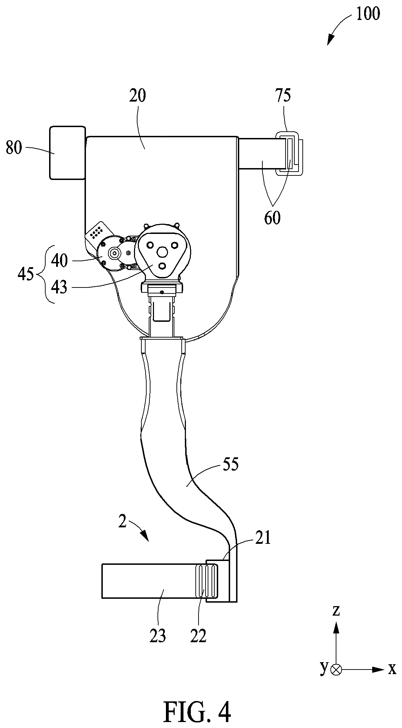

FIG. 3 illustrates a front surface of a wearable device according to an embodiment, and FIG. 4 illustrates a side of a wearable device according to an embodiment.

Referring to FIGS. 3 and 4, a wearable device 100 worn on a body of a user according to an embodiment may include the housing 80, the waist support frames 20 and 25, the driving modules 35 and 45, the leg support frames 50 and 55, thigh fastening portions 1 and 2, and a waist fastening portion. The waist fastening portion may include a belt 60 and an auxiliary belt 75. In an embodiment, at least one of the above components (e.g., the auxiliary belt 75) may be omitted from the wearable device 100, or one or more other components (e.g., a fastening sensing module for sensing fastening of the thigh fastening portions 1 and 2 or the waist fastening portion) may be added to the wearable device 100.

In an embodiment, in the housing 80, a control module (not shown) (e.g., the control module 130 of FIG. 1, the control module 510 of FIGS. 5A and 5B, an IMU (not shown) (e.g., the IMU 135 of FIG. 1), and a battery (not shown) may be disposed. The housing 80 may protect the control module, the IMU, and the battery. For example, the housing 80 may be disposed on a back of the user or a back side of a waist of the user, based on a state in which the wearable device 100 is worn on the body of the user. The control module may generate a control signal to control the wearable device 100 and may control an operation of the wearable device 100 based on the control signal. The control module may include a control circuit including a processor (comprising processing circuitry), a memory, and/or a communication module to control actuators 30 and 40 of the driving modules 35 and 45. The control module may further include a power supply module (not shown) to supply power from a battery to each of the components of the wearable device 100.

In an embodiment, the wearable device 100 includes a sensor module (not shown) (e.g., the sensor module 520 of FIG. 5A), comprising at least one sensor, (where use of only one sensor is not part of the claimed invention) configured to obtain sensor data from at least one sensor. The sensor module may obtain sensor data that changes according to a motion of a user. In an embodiment, the sensor module may obtain sensor data including motion information of the user in a physical ability measurement mode of the user. The sensor module may include, for example, an IMU (e.g., the IMU 135 of FIG. 1) to measure an upper body motion value of a user, and an angle sensor (e.g., the angle sensor 125 of FIG. 1) to measure a hip joint angle value of the user, but is not limited thereto. For example, the sensor module may further include at least one of a position sensor, a temperature sensor, a biosignal sensor, and/or a proximity sensor.

In an embodiment, the waist support frames 20 and 25 support a part of the body of the user when the wearable device 100 is worn on the body of the user. The waist support frames 20 and 25 may contact at least a portion of an outer surface of the user. The waist support frames 20 and 25 may be formed to be curved in a shape corresponding to a contact portion of the body of the user. For example, the waist support frames 20 and 25 may have a shape that wraps around an outer surface of the waist (or a pelvis) of the user, and support the waist or the pelvis of the user. The waist support frames 20 and 25 may include a first waist support frame 25 to support a right side of the waist of the user, and a second waist support frame 20 to support a left side of the waist of the user. The waist support frames 20 and 25 may be connected, directly or indirectly, to the housing 80.

The waist fastening portion may be connected, directly or indirectly, to the waist support frames 20 and 25 to fix the waist support frames 20 and 25 to the waist of the user. The waist fastening portion may include, for example, a pair of belts 60 and an auxiliary belt 75. The auxiliary belt 75 may be connected, directly or indirectly, to one of the pair of belts 60.

In an embodiment, the pair of belts 60 may be connected, directly or indirectly, to the waist support frames 20 and 25. In a state in which the user does not wear the wearable device 100, the pair of belts 60 may maintain a shape of extending forward (in a +x direction) and may not hinder the user from wearing the pair of waist support frames 20. In a state in which the user wears the pair of waist support frames 20 and 25, the pair of belts 60 may be transformed to enclose a front portion of the user. The waist support frames 20 and 25 and the pair of belts 60 may entirely wrap around a circumference of the waist of the user. In an embodiment, the auxiliary belt 75 may fix the pair of belts 60 to each other in a state in which the belts 60 overlap each other. For example, one of the pair of belts 60 may wrap around the other belt together with the auxiliary belt 75.

The driving modules 35 and 45 may generate an external force (e.g., torque) to be applied to the body of the user based on the control signal generated by the control module. For example, the driving modules 35 and 45 may generate an external force to be applied to a leg of the user under the control of the control module. In an embodiment, the driving modules 35 and 45 may include a first driving module 45 disposed in a position corresponding to a position of a right hip joint of the user, and a second driving module 35 disposed in a position corresponding to a position of a left hip joint of the user. The first driving module 45 may include a first actuator 40 and a first joint member 43, and the second driving module 35 may include a second actuator 30 and a second joint member 33. The first actuator 40 may provide power to be transmitted to the first joint member 43, and the second actuator 30 may provide power to be transmitted to the second joint member 33. The first actuator 40 and the second actuator 30 may each include a motor configured to generate power (e.g., torque) by receiving power from a battery. When the motor is driven by receiving a power supply, the motor may provide a force (e.g., an assistance force) to assist a body motion of the user, or a force (e.g., resistance force) to hinder the body motion. In an embodiment, the control module may adjust an intensity and a direction of the force generated by the motor, by adjusting voltage and/or current supplied to the motor.

In an embodiment, the first joint member 43 and the second joint member 33 may receive power from the first actuator 40 and the second actuator 30, respectively, and may apply an external force to the body of the user based on the received power. The first joint member 43 and the second joint member 33 may be disposed at positions corresponding to joint portions of the user, respectively. The first joint member 43 and the second joint member 33 may be disposed on one side of the waist support frame 25 and one side of the waist support frame 20, respectively. One side of the first joint member 43 may be connected, directly or indirectly, to the first actuator 40, and another side of the first joint member 43 may be connected, directly or indirectly, to a first leg support frame 55. The first joint member 43 may be rotated by the power received from the first actuator 40. An encoder or a hall sensor that may operate as an angle sensor to measure a rotation angle (corresponding to a joint angle of the user) of the first joint member 43 may be disposed on one side of the first joint member 43. One side of the second joint member 33 may be connected, directly or indirectly, to the second actuator 30, and another side of the second joint member 33 may be connected, directly or indirectly, to a second leg support frame 50. The second joint member 33 may be rotated by the power received from the second actuator 30. An encoder or a hall sensor that may operate as an angle sensor to measure a rotation angle of the second joint member 33 may be disposed on one side of the second joint member 33.

In an embodiment, the first actuator 40 may be disposed in a lateral direction of the first joint member 43, and the second actuator 30 may be disposed in a lateral direction of the second joint member 33. A rotation axis of the first actuator 40 and a rotation axis of the first joint member 43 may be spaced apart from each other, and a rotation axis of the second actuator 30 and a rotation axis of the second joint member 33 may also be spaced apart from each other. However, the embodiments are not limited thereto, and the actuator(s) 30, 40 and the joint member(s) 33, 43 may also share a rotation axis. In an embodiment, the actuators 30 and 40 may be spaced apart from the joint members 33 and 43, respectively. In this case, each driving module 35, 45 may further include a power transmission module (not shown) configured to transfer power from the actuators 30, 40 to the joint members 33, 43. The power transmission module may be a rotary body, such as a gear, or a longitudinal member, such as a wire, a cable, a string, a spring, a belt, or a chain. However, the scope of embodiments is not limited by the above-described position relationship between the actuator(s) 30, 40 and the joint member(s) 33, 43 and a power transmission structure.

In an embodiment, the leg support frame 50, 55 supports a leg (e.g., a thigh) of the user when the wearable device 100 is worn on the leg of the user. For example, the leg support frames 50, 55 may transmit power generated by the driving modules 35, 45 to a thigh of the user, and the power may act as an external force to be applied to a motion of the leg of the user. One end portion of each leg support frame 50, 55 may be connected, directly or indirectly, to the corresponding joint member 33, 43 to be rotated, and another end portion of each leg support frame 50, 55 may be connected, directly or indirectly, to a corresponding cover 11, 21 of the corresponding thigh fastening portion 1, 2. Accordingly, the leg support frames 50, 55 may transmit the power generated by the driving modules 35, 45 to the thighs of the user while supporting the thighs of the user. For example, the leg support frames 50, 55 may push and/or pull the thighs of the user. The leg support frames 50, 55 may extend in a longitudinal direction of the thighs of the user. The leg support frames 50, 55 may be bent to wrap around at least a portion of a circumference of the respective thighs of the user. For example, an upper portion of the leg support frames 50, 55 may cover a portion of the body of the user facing a side (+y direction or - y direction), and a lower portion of the leg support frames 50, 55 may cover a portion of the body of the user facing the front portion (+x direction). The leg support frames 50 and 55 may include the first leg support frame 55 to support a right leg of the user, and the second leg support frame 50 to support a left leg of the user.

The thigh fastening portions 1 and 2 may be connected, directly or indirectly, to the leg support frames 50 and 55 and may fix the leg support frames 50 and 55 to the thigh. The thigh fastening portions 1 and 2 may include a first thigh fastening portion 2 to fix the first leg support frame 55 to a right thigh of the user, and a second thigh fastening portion 1 to fix the second leg support frame 50 to a left thigh of the user. The first thigh fastening portion 2 may include a first cover 21, a first fastening frame 22, and a first strap 23. The second thigh fastening portion 1 may include a second cover 11, a second fastening frame 12, and a second strap 13.

In an embodiment, the covers 11, 21 may apply torque generated by the respective driving modules 35, 45 to the respective thighs of the user. For example, each of the covers 11, 21 may be disposed on one side of the respective thigh of the user to push and/or pull the thigh of the user. The covers 11, 21 may be disposed on a front surface of the thigh of the user. The covers 11, 21 may be disposed in a circumferential direction of the thigh of the user. The covers 11, 21 may extend toward both sides around the other end portion of the leg support frames 50, 55, and may include a curved surface corresponding to or related to the thigh of the user. One end of each cover 11, 21 may be connected, directly or indirectly, to the corresponding fastening frame 12, 22, and another end of each cover 11, 21 may be connected, directly or indirectly, to the corresponding strap 13, 23.

In an embodiment, one end of each fastening frame 12, 22 may be connected, directly or indirectly, to one side of the respective cover 11, 21, and another end of each fastening frame 12, 22 may be connected, directly or indirectly, to the corresponding strap 13, 23. For example, each fastening frame 12, 22 may be disposed to enclose at least a portion of the circumference of the corresponding thigh of the user to prevent or reduce the chance of the thigh of the user from being separated from the corresponding leg support frame 50, 55. The first fastening frame 22 may have a fastening structure that connects the first cover 21 and the first strap 23, and the second fastening frame 12 may have a fastening structure that connects the second cover 11 and the second strap 13.

The straps 13, 23 may enclose parts other than parts covered by the covers 11, 21 and the fastening frames 12, 22 in the circumference of the thigh of the user, and may include an elastic material (e.g., a band).

In an embodiment, the wearable device 100 may individually support a proximal part and a distal part of the user, to assist in a relative movement between the proximal part and the distal part. Among the components of the wearable device 100, components worn on the proximal part of the user may be referred to as "proximal wearing units," and components worn on the distal part may be referred to as "distal wearing units". For example, among the components of the wearable device 100, the housing 80, the waist support frames 20 and 25, the pair of belts 60, and the auxiliary belt 75 may correspond to a proximal wearing unit, and the thigh fastening portions 1 and 2 may correspond to a distal wearing unit. For example, the proximal wearing unit may be worn on a waist or a pelvis of the user, and the distal wearing unit may be worn on a thigh or a calf of the user. Positions in which the proximal wearing unit and the distal wearing unit are worn are not limited thereto. For example, the proximal wearing unit may be worn on a torso or a shoulder of the user, and the distal wearing unit may be worn on an upper arm or a lower arm of the user.

FIGS. 5A and 5B are diagrams illustrating a configuration of a control system of a wearable device according to an embodiment.

Referring to FIG. 5A, a wearable device (e.g., the wearable device 100) may be controlled by a control system 500. The control system 500 may include the control module 510, the sensor module 520, a driving module 530, and a battery 540. The driving module 530 may include a motor 534 to generate power (e.g., torque), and a motor driver circuit 532 to drive the motor 534. FIG. 5A illustrates a single sensor module 520, and the driving module 530 including a single motor driver circuit 532 and a single motor 534, however, this is merely an example. Referring to FIG. 5B, two or more sensor modules (e.g., sensor modules 520 and 520-1), two or more motor driver circuits (e.g., motor driver circuits 532 and 532-1), and two or more motors (e.g., motors 534 and 534-1) may be provided in a similar control system 500-1. The driving module 530 including the motor driver circuit 532 and the motor 534 may correspond to the first driving module 45 of FIG. 3, and a driving module 530-1 including the motor driver circuit 532-1 and the motor 534-1 may correspond to the second driving module 35 of FIG. 3. The following description of each of the sensor module 520, the motor driver circuit 532, and the motor 534 may also be applied to the sensor module 520-1, the motor driver circuit 532-1, and the motor 534-1 shown in FIG. 5B.

Referring back to FIG. 5A, the sensor module 520 may include at least one sensor (this is not part of the invention, which requires at least two sensors in the sensor module). The sensor module 520 obtains sensor data that changes according to a motion of a user. The sensor module includes sensor data including motion information of the user in a physical ability measurement mode of the user. The sensor module 520 may transmit the obtained sensor data to the control module 510. The sensor module 520 includes an IMU, an angle sensor (e.g., an encoder, and a hall sensor), and may include position sensor, a proximity sensor, a biosignal sensor, and a temperature sensor. The IMU may measure an upper body motion value of the user. For example, the IMU may sense accelerations of an X-axis, a Y-axis, and a Z-axis and angular velocities of the X-axis, Y-axis, and Z-axis according to a motion of the user. The angle sensor measures a hip joint angle value according to a motion of a leg of the user. Sensor data that may be measured by the angle sensor may include, for example, a hip joint angle value of a right le.g., a hip joint angle value of a left leg, and information on a direction of a motion of a leg.

The battery 540 may supply power to each component of the wearable device. The wearable device may convert power of the battery 540 according to an operating voltage of each component of the wearable device and supply the power to each component.

The driving module 530 may generate an external force to be applied to a leg of the user under the control of the control module 510. The driving module 530 may be disposed in a position corresponding to a position of a hip joint of the user and may generate torque to be applied to a leg of the user based on a control signal generated by the control module 510. The control module 510 may transmit the control signal to the motor driver circuit 532, and the motor driver circuit 532 may generate a current signal corresponding to the control signal and supply the current signal to the motor 534, to control an operation of the motor 534. The current signal may not be supplied to the motor 534 according to the control signal. If the motor 534 is driven by receiving the current signal, the motor 534 may generate a force to assist in a leg motion of the user or torque to hinder the leg motion.

The control module 510 may control an overall operation of the wearable device, and may generate a control signal to control each component (e.g., the driving module 530). The control module 510 may include a processor 512, a memory 514, and a communication module 516.

The processor 512 may execute, for example, software to control at least one other component (e.g., a hardware or software component) of the wearable device connected, directly or indirectly, to the processor 512, and may perform a variety of data processing or computation. According to an embodiment, as at least a part of data processing or computation, the processor 512 may store instructions or data received from another component (e.g., the communication module 516, comprising communication circuitry) in the memory 514, may process the instructions or the data stored in the memory 514, and may store result data in the memory 514. According to an embodiment, the processor 512 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of, or in conjunction with the main processor. The auxiliary processor may be implemented separately from the main processor or as a part of the main processor.

The memory 514 may store a variety of data used by at least one component (e.g., the processor 512) of the control module 510. The variety of data may include, for example, software, sensor data, input data or output data for instructions related thereto. The memory 514 may include a volatile memory or a non-volatile memory (e.g., a random-access memory (RAM), a dynamic RAM (DRAM), or a static RAM (SRAM)).

The communication module 516, comprising communication circuitry, may support establishing a direct (e.g., wired) communication channel or a wireless communication channel between the control module 510 and another component of the wearable device or an external electronic device (e.g., the electronic device 210 or the other wearable devices 220 of FIG. 2), and performing communication via the established communication channel. The communication module 516 transmits the sensor data obtained by the sensor module 520 to an external electronic device (e.g., the electronic device 210 of FIG. 2) and receive a control signal from the electronic device. The communication module 516 may include one or more CPs that are operable independently of the processor 512 and that support a direct (e.g., wired) communication or a wireless communication. According to an embodiment, the communication module 516 may include a wireless communication module (e.g., a cellular communication module, a short-range wireless communication module, or a global navigation satellite system (GNSS) communication module), and/or a wired communication module. A corresponding one of these communication modules may communicate with another component of the wearable device and/or an external electronic device via a short-range communication network, such as Bluetooth^{™}, wireless-fidelity (Wi-Fi), an ANT+ or infrared data association (IrDA), or a long-range communication network, such as a legacy cellular network, a 5G network, a next-generation communication network, the Internet, or a computer network (e.g., a local area network (LAN) or a wide region network (WAN).

The wearable device operates in a physical ability measurement mode to measure a physical ability of the user. The communication module 516 may receive a control signal related to a progress of the physical ability measurement mode from the electronic device, and transmit the received control signal to the processor 512. The sensor module 520 may generate sensor data including physical ability information of the user and store the generated sensor data in the memory 514. The sensor data may include sensor values, output from a predetermined sensor (e.g., an angle sensor, and an IMU) included in the sensor module 520, over time. The communication module 51 transmits to the electronic device, sensor data obtained from the sensor module 520 during the physical ability measurement mode.

The processor 512, comprising processing circuitry, controls the communication module 516 and the sensor module 520 to operate the physical ability measurement mode. When a control signal for a measurement of a target physical ability (e.g., a walking ability, a muscular strength, and a balance ability) of the user is received from the electronic device, the processor 512 may activate the physical ability measurement mode in response to the control signal. The processor 512 controls the sensor module 520 to obtain sensor data in the physical ability measurement mode. When the physical ability measurement mode is activated, the processor 512 initializes (or calibrates) the sensor data output from the sensor module 520, and controls a recording of the sensor data to be started when a predetermined period of time for the measurement of the target physical ability elapses. A process of initializing the sensor data may be a process of matching the sensor data output from the sensor module 520 to a reference value when the user takes a ready posture to measure the physical ability. Since different users may have different body states (e.g., a leg angle, and a torso inclination) in the ready posture, the process of initializing the sensor data output from the sensor module 520 may be required to accurately measure the physical ability in the ready posture of the user.

After the process of initializing the sensor data is performed, the sensor module 520 obtains sensor data according to a motion of the user. The sensor module 520 obtains first sensor data including motion information of the user measured by the IMU, and second sensor data including a hip joint angle of the user measured by the angle sensor. The processor 512 controls the communication module 516 to transmit the first sensor data and the second sensor data to the electronic device 210 in the physical ability measurement mode, so that the electronic device 210 may calculate (e.g., determine) a temporal gait index based on the first sensor data, calculate (e.g., determine) a spatial gait index based on the second sensor data, and calculate (e.g., determine) physical ability information of the user based on the temporal gait index and the spatial gait index. The physical ability information may be provided to the user through a program executed in the electronic device.

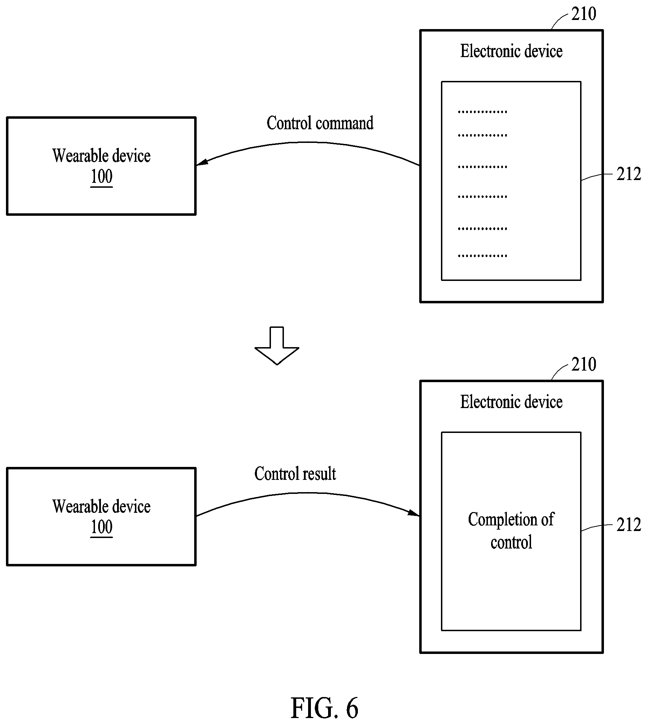

FIG. 6 is a diagram illustrating an interaction between a wearable device and an electronic device according to an embodiment.

Referring to FIG. 6, the wearable device 100 communicates with the electronic device 210. For example, the electronic device 210 may be a user terminal of a user who uses the wearable device 100, or an exclusive controller for the wearable device 100. According to an embodiment, the wearable device 100 and the electronic device 210 may be connected using a short-range wireless communication scheme.

The electronic device 210 may display a user interface (UI) screen for controlling an operation of the wearable device 100 or for a measurement of a physical ability of a user on the display 212. In an embodiment, the user may input a command (e.g., a command to execute a physical ability measurement mode) to control the operation of the wearable device 100 through the UI screen on the display 212 of the electronic device 210. The electronic device 210 may generate a control command corresponding to the command and transmit the generated control command to the wearable device 100. The wearable device 100 may operate according to the received control command and transmit a control result and/or measured data (e.g., sensor data) to the electronic device 210. The electronic device 210 may provide the control result and/or result information (e.g., physical ability information) derived by analyzing data of the wearable device 100 to the user through the display 212.

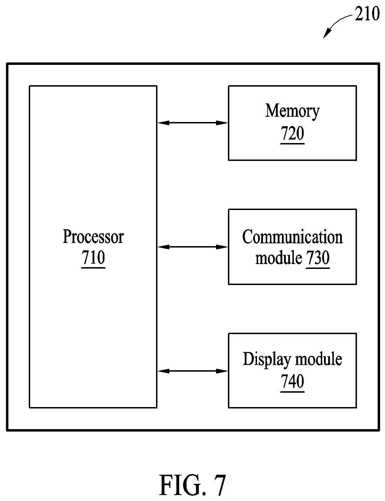

FIG. 7 is a diagram illustrating a configuration of an electronic device according to an embodiment.

Referring to FIG. 7, the electronic device 210 may include a processor 710, a memory 720, a communication module 730, and a display module 740. In some embodiments, one or more other components (e.g., an input module, and a sensor module) may be added to the electronic device 210.

The processor 710 may control at least one other component (e.g., a hardware or software component) of the electronic device 210 connected to the processor 710, and may perform a variety of data processing or computation. According to an embodiment, as at least a part of data processing or computation, the processor 710 may store instructions or data received from another component (e.g., the communication module 730) in the memory 720, may process the instructions or the data stored in the memory 720, and may store result data in the memory 720.

According to an embodiment, the processor 710 may include a main processor (e.g., a CPU or an AP) or an auxiliary processor (e.g., a GPU, an NPU, an ISP, a sensor hub processor, or a CP) that is operable independently of, or in conjunction with the main processor.

The memory 720 may store a variety of data used by at least one component (e.g., the processor 710 or the communication module 730) of the electronic device 210. The data may include, for example, a program (e.g., an application), and input data or output data for a command related thereto. The memory 720 may include at least one instruction executable by the processor 710. The memory 720 may include a volatile memory or a non-volatile memory.

The communication module 730 may support establishing a direct (e.g., wired) communication channel or a wireless communication channel between the electronic device 210 and another electronic device (e.g., the wearable device 100, the other wearable devices 222, 224, 226 in 220, and the server 230 in Fig. 2), and performing communication via the established communication channel. The communication module 730 may include one or more CPs that are operable independently of the processor 710 (e.g., an AP) and that support a direct (e.g., wired) communication or a wireless communication. According to an embodiment, the communication module 730 may include a wireless communication module (e.g., a Bluetooth communication module, a cellular communication module, a short-range wireless communication module, or a GNSS communication module) that performs wireless communication, or a wired communication module (e.g., a LAN communication module, or a power line communication (PLC) module). A corresponding one of these communication modules may communicate with another electronic device via a first network (e.g., a short-range communication network, such as Bluetooth^{™}, Wi-Fi direct, or IrDA) or a second network (e.g., a long-range communication network, such as a legacy cellular network, a 5G network, a next-generation communication network, the Internet, or a computer network (e.g., a LAN or a WAN). For example, the communication module 730 may receive sensor data including motion information of a user wearing a wearable device from the wearable device.

The display module 740 may visually provide information to the outside (e.g., a user) of the electronic device 210. The display module 740 may include, for example, a display, a hologram device, or a projector and control circuitry to control a corresponding one of the display, the hologram device, and the projector. For example, the display module 740 may include a touch sensor adapted to sense a touch, or a pressure sensor adapted to measure an intensity of a force incurred by the touch. In an embodiment, the display module 740 may output a UI screen for guiding a measurement of a physical ability of the user, and may output physical ability information of the user.

In an embodiment, the electronic device 210 may operate a physical ability measurement mode for measuring the physical ability of the user, by interworking with the wearable device 100. The user may instruct the electronic device 210 to execute the physical ability measurement mode through a user input, and may select a physical ability measurement test (e.g., a walking ability measurement, a TUG test, a 5×STS test, and an SPPB test) for a target physical ability to be measured. When a user input for a measurement of the target physical ability of the user is received, the processor 710 may control the communication module 730 to transmit a control signal for the measurement of the target physical ability to the wearable device in response to the user input. When the control signal for the measurement of the target physical ability is received from the electronic device, the wearable device activates the physical ability measurement mode. The wearable device obtains sensor data including motion information of the user in the physical ability measurement mode. The wearable device transmits the obtained sensor data to the electronic device 210. In an embodiment, the processor 710 may provide a UI for guiding the measurement of the target physical ability to the user through the display module 740.

The processor 710 may receive the sensor data from the wearable device through the communication module 730 and calculate physical ability information of the user based on the received sensor data. The processor 710 may extract a feature point corresponding to a reference measurement value for the measurement of the target physical ability from the sensor data, and may estimate the physical ability of the user based on the extracted feature point. One or more feature points may be extracted from the sensor data. The processor 710 may generate physical ability information on the estimated physical ability.

In an embodiment, when the user selects a walking ability measurement test from among physical ability measurement tests provided by the electronic device 210, the processor 710 may extract first feature points corresponding to a heel strike and second feature points corresponding to a toe-off from a motion value of the user measured by an IMU of the wearable device. The processor 710 may estimate a gait index of the user based on the first feature points and the second feature points. The gait index that may be estimated by the processor 710 may include, for example, a gait speed, a gait time period, a stride length, a gait symmetry index, and a gait fluctuation index.

Each embodiment herein may be used in combination with any other embodiment(s) described herein.

In an embodiment, the processor 710 may calculate a temporal gait index based on first sensor data including motion information of the user measured by an inertial sensor of the wearable device 100. The processor 710 may estimate (or calculate) the temporal gait index based on a time interval between first feature points temporally adj acent to each other among the first feature points, a time interval between second feature points temporally adjacent to each other among the second feature points, and a time interval between a first feature point and a second feature point temporally adjacent to the first feature point. The temporal gait index may include, for example, at least one of a step time, a swing time, a stance time, a stride time, or a double support time of the user.

In an embodiment, the processor 710 may calculate a spatial gait index based on second sensor data including a hip joint angle of the user measured by an angle sensor of the wearable device 100. The processor 710 may estimate (or calculate) the spatial gait index based on a hip joint angle value at a point in time corresponding to a heel strike from a hip joint angle value (e.g., a hip joint angle value corresponding to a right leg, and a hip joint angle value corresponding to a left leg) of the user measured by an angle sensor of the wearable device. The spatial gait index may include, for example, at least one of a step length, a stride length, or a leg length of the user.

In an embodiment, the processor 710 may estimate at least one of a gait speed, a gait fluctuation index, or a gait symmetry index of the user, based on the temporal gait index and the spatial gait index. The processor 710 may determine a sum of step lengths and a total period of walking time, which are estimated in a walking interval, and may estimate an average gait speed of the user by dividing the sum of the step lengths by the total period of walking time.

In an embodiment, the processor 710 may calculate an average value of left step lengths and an average value of right step lengths for multiple strides of the user, and determine a gait symmetry index based on a difference between the average value of the left step lengths and the average value of the right step lengths. The processor 710 may determine the gait symmetry index based on a difference between a left step time and a right step time for multiple strides of the user.

In an embodiment, the processor 710 may determine a gait fluctuation index for a gait motion of the user based on a standard deviation of stride lengths measured in a predefined number of steps (e.g., "10" steps, and "20" steps) during the gait motion of the user or a standard deviation of stride times measured in the predefined number of steps. For example, the processor 710 may determine, as a gait fluctuation index, a value obtained by dividing the standard deviation of the stride lengths by an average of the stride lengths, or a value obtained by diving the standard deviation of the stride times by an average of the stride times.

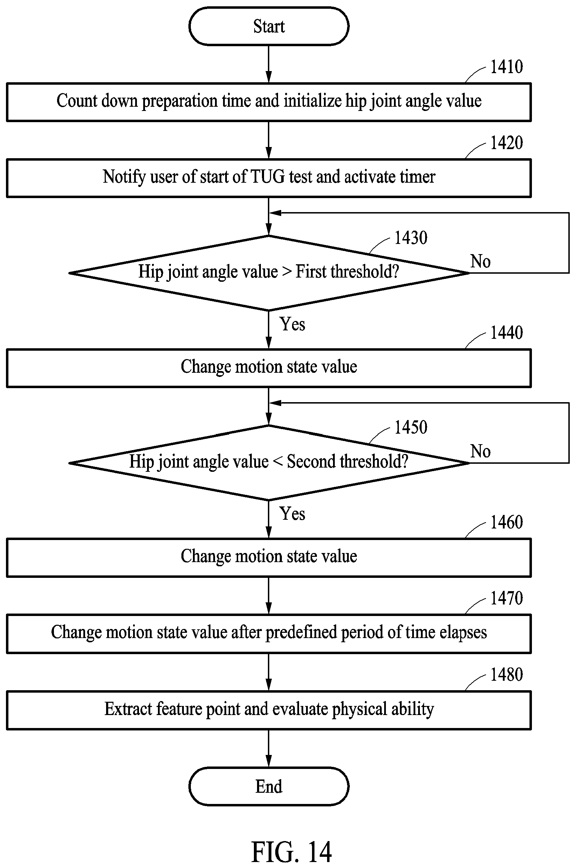

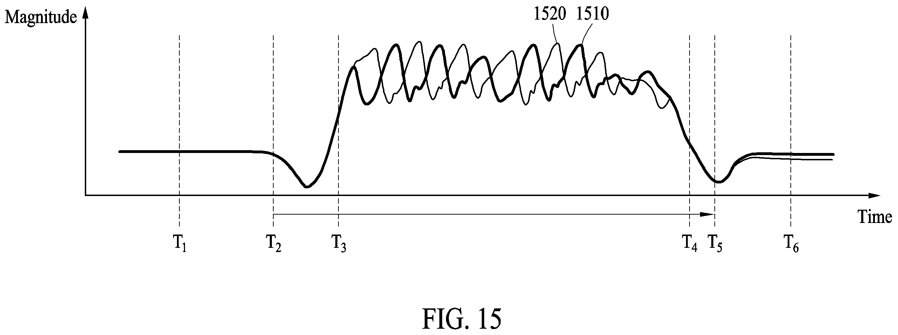

In an embodiment, the user may select a TUG test from among the physical ability measurement tests provided by the electronic device 210. In the TUG test, a period of time, in which the user wearing the wearable device sits in a chair, walks a predetermined distance (e.g., three meters (m)) from the chair to return to the chair, and sits again in the chair, may be measured. Based on the measured period of time, the physical ability (e.g., a mobility, a balance ability, a walking ability, a fall risk, etc.) of the user may be estimated. In an example in which the physical ability measurement mode for measuring the target physical ability is the TUG test, when a hip joint angle value included in the sensor data is greater than or equal to a first threshold, the processor 710 may recognize that the user has risen from the chair. When the hip joint angle value is less than or equal to a second threshold, the processor 710 may recognize that the user sits in the chair and may extract, as a feature point, a hip joint angle value corresponding to a minimum/low value among hip joint angle values measured during a predefined period of time (e.g., two seconds) that elapses from a point in time at which it is recognized that the user sits in the chair. The processor 710 may calculate physical ability information of the user based on a period of time from a start of the TUG test to a point in time the hip joint angle value corresponding to the minimum/low value is extracted. The measurement of the physical ability of the user through the TUG test will be described in more detail with reference to FIGS. 14 and 15.

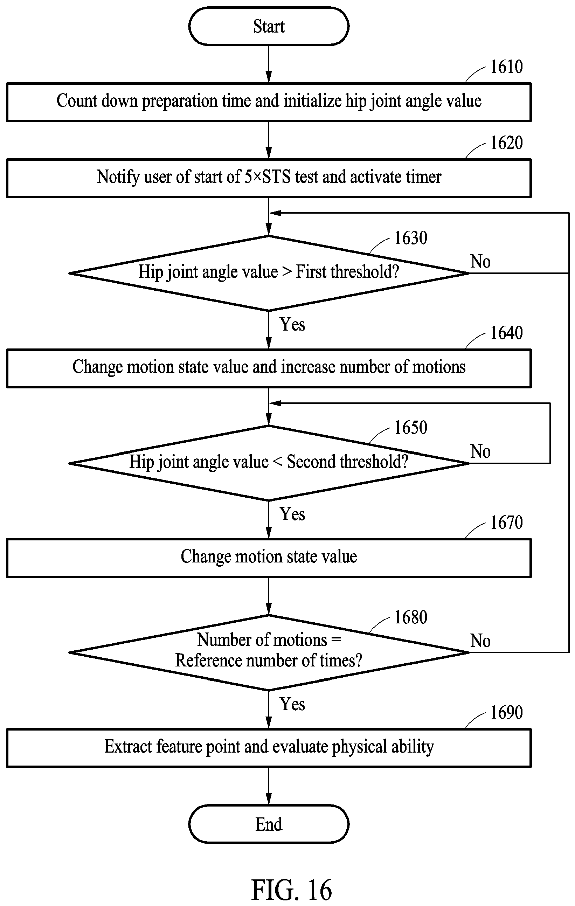

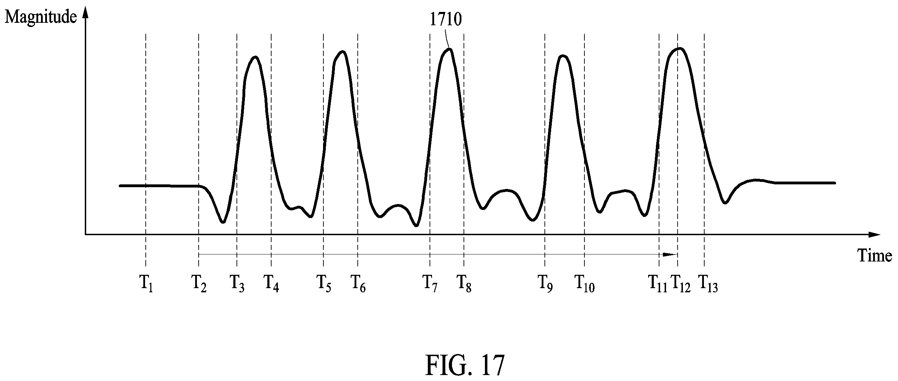

In an embodiment, the user may select a 5×STS test from among the physical ability measurement tests provided by the electronic device 210. In the 5×STS test, a period of time, in which the user wearing the wearable device performs five times a motion of sitting in a chair, rising from the chair, and sitting in the chair again, may be measured. Based on the measured period of time, the physical ability (e.g., a leg muscle strength) of the user may be estimated. In an example in which the physical ability measurement mode for measuring the target physical ability is the 5×STS test, when a hip joint angle value (e.g., hip joint angle values of both legs) included in the sensor data is greater than or equal to the first threshold, the processor 710 may recognize that the user has risen from the chair and may increase a number of times the user rises from the chair. When the hip joint angle value is less than or equal to the second threshold, the processor 710 may recognize that the user sits in the chair. When the number of times the user rises from the chair reaches a reference number of times, the processor 710 may extract, as a feature point, a hip joint angle value that is a maximal or large value appearing immediately before when the number of times the user rises from the chair reaches the reference number of times. The processor 710 may calculate physical ability information of the user based on a period of time from a start of the 5×STS test to a point in time at which a corresponding feature point is extracted. The measurement of the physical ability of the user through the 5×STS test will be described in more detail with reference to FIGS. 16 and 17.

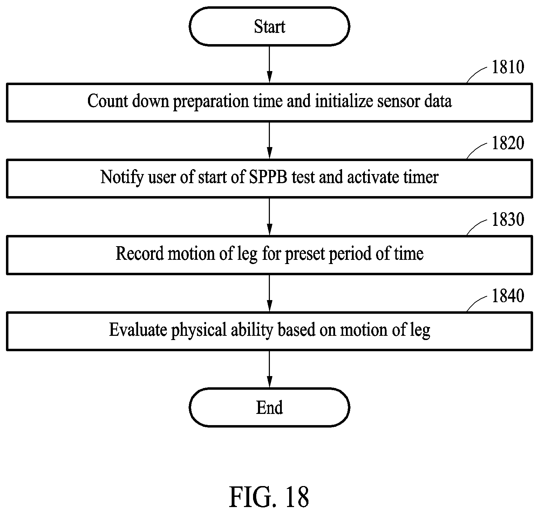

In an embodiment, the user may select an SPPB test from among the physical ability measurement tests provided by the electronic device 210. In the SPPB test, a foot motion and an upper body motion of the user wearing the wearable device in a state of standing in a predetermined leg posture may be measured for a defined period of time. The physical ability (e.g., a balance ability) of the user may be estimated based on at least one of whether a foot moves for a corresponding period of time, a time in which the foot motion occurs, whether an upper body moves for the period of time, or a time in which the upper body motion occurs. In an example in which the physical ability measurement mode for measuring the target physical ability is the SPPB test, when a hip joint angle value measured for one time interval (e.g., ten seconds) is greater than or equal to the first threshold, the processor 710 may determine that a leg of the user moves. When a value of an upper body motion of the user measured for one time interval is greater than or equal to the second threshold, the processor 710 may determine that an upper body of the user moves. The processor 710 may calculate physical ability information of the user based on a posture (e.g., a side-by-side stance in which feet are aligned side by side, a tandem stance in which both feet are aligned in a line, and a semi-tandem stance in which one foot is placed in front of the other foot by half of the other foot) of the user on which the SPPB test is to be performed, a value of an upper body motion measured for one time interval, and a time in which it is determined that a leg moves. The processor 710 may assign different scores for each of a posture of the user, a value of an upper body motion of the user, and a time in which a leg moves, and may determine a final score for the SPPB test of the user based on scores for each evaluation factor. The measurement of the physical ability of the user through the SPPB test will be described in more detail with reference to FIG. 18. Each "processor" herein comprises processing circuitry.

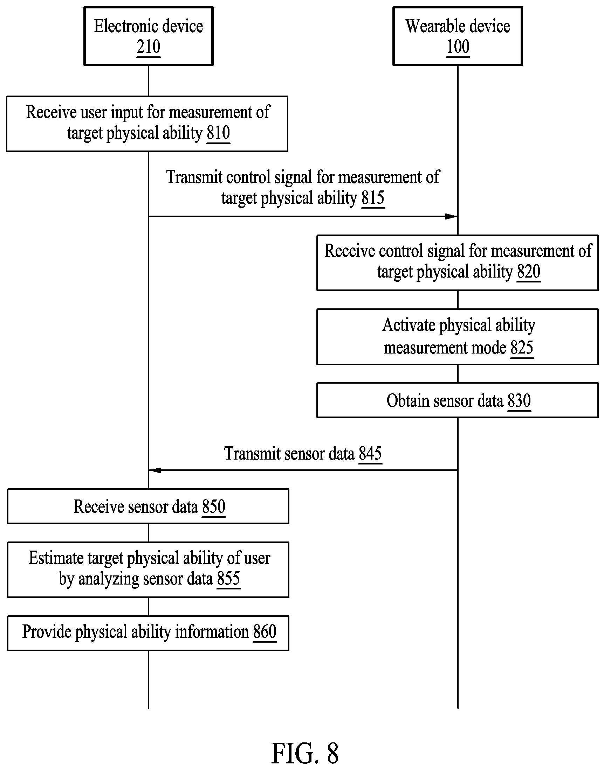

FIG. 8 is a diagram illustrating an operating method of an electronic device and a wearable device to measure a physical ability of a user according to an embodiment. In an embodiment, at least one of operations of FIG. 8 may be performed concurrently or in parallel with other operations, and an order of the operations may be changed. In addition, at least one of the operations may be omitted, or another operation may be additionally performed.

Referring to FIG. 8, in operation 810, the electronic device 210 may receive a user input for a measurement of a target physical ability of a user. The user may select a physical ability measurement test to be performed on an application executed in the electronic device 210. In an embodiment, the user may set an evaluation factor (e.g., a movement distance in a walking ability measurement test and a TUG test, a number of times a user stands and sits in a chair in a 5×STS test, and a type of leg postures in an SPPB test) of a predetermined physical ability measurement test, using the application. Information on the set evaluation factor may be transmitted to the wearable device 100.

In operation 815, the electronic device 210 may transmit a control signal for the measurement of the target physical ability to the wearable device 100 in response to the user input in operation 810. In operation 820, the wearable device 100 receives the control signal for the measurement of the target physical ability of the user from the electronic device 210. For example, the wearable device 100 may receive information on a type of a physical ability measurement test to be performed and an evaluation factor set by the user for a selected physical ability measurement test from the electronic device 210.

In operation 825, the wearable device 100 activates a physical ability measurement mode in response to the control signal received in operation 820. In operation 830, the wearable device 100 obtains sensor data (e.g., a hip joint angle value, and an upper body motion value) including motion information of the user, using a sensor module of the wearable device 100 in the physical ability measurement mode. For example, the wearable device 100 may not generate torque through a driving module when operating in the physical ability measurement mode.

In operation 845, the wearable device 100 transmits the obtained sensor data to the electronic device 210 so that the electronic device 210 may estimate the physical ability of the user based on the sensor data and provide the user with physical ability information on the estimated physical ability.

In operation 850, the electronic device 210 receives the sensor data including the motion information of the user for the measurement of the target physical ability from the wearable device 100. The electronic device 21 receives first sensor data including motion information of the user measured by an inertial sensor of the wearable device 100 and second sensor data including a hip joint angle of the user measured by an angle sensor of the wearable device 100 from the wearable device 100.

In operation 855, the electronic device 210 may estimate the target physical ability of the user by analyzing the sensor data. The electronic device 210 may calculate physical ability information on the target physical ability of the user based on the first sensor data and the second sensor data. The electronic device 210 may calculate a temporal gait index based on the first sensor data and calculate a spatial gait index based on the second sensor data. In addition, the electronic device 210 may calculate the physical ability information based on the temporal gait index and the spatial gait index. For example, the electronic device 210 may extract a feature point corresponding to a reference measurement value for the measurement of the target physical ability from the sensor data, and may estimate the target physical ability of the user based on the extracted feature point. In an embodiment, the electronic device 210 may evaluate the target physical ability of the user based on a period of time from a start of the physical ability measurement test to a point in time at which the feature point is extracted, or a time interval between feature points. For example, in the case of the TUG test or the 5×STS test, it may be determined that if a period of time until a feature point is extracted decreases, the physical ability of the user increases.

In operation 860, the electronic device 210 may provide the user with physical ability information on the target physical ability estimated in operation 855. The user may check the physical ability information using the application executed in the electronic device 210. In an embodiment, the electronic device 210 may recommend an exercise program suitable for improving an insufficient physical ability to the user, based on the physical ability information of the user.

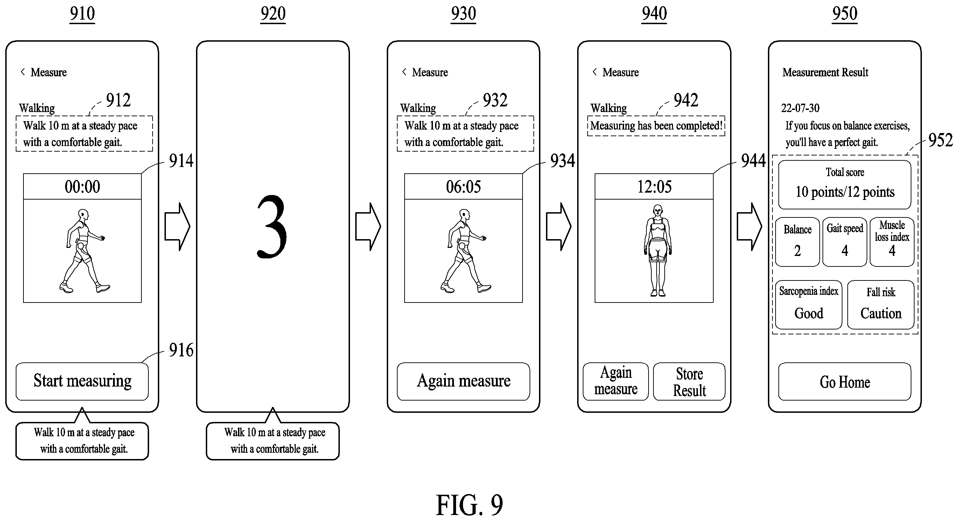

FIG. 9 is a diagram illustrating a UI provided to a user through an electronic device to measure a walking ability, according to an embodiment.

FIG. 9 illustrates UI screens provided to the user through an application of the electronic device (e.g., the electronic device 210) when the user selects a walking ability measurement test. Before the walking ability measurement test, the user may mark a reference line with a predetermined distance (e.g., 10 m) based on a start point and an end point on the ground. The user may stand on the start point while wearing a wearable device (e.g., the wearable device 100). Until the user stands at the start point, a UI screen 910 may be displayed on the electronic device. The UI screen 910 may include a description 912 of a walking ability measurement scheme, a time counter 914, and a selection icon 916 to control a start of a measurement, however, a configuration of the UI screen 910 is limited thereto.

If a preparation for a measurement of a walking ability is completed, the user may send a request for a start of the walking ability measurement test by touching the selection icon 916 on the UI screen 910. Subsequently, the electronic device may display a UI screen 920 for counting down a preparation time. During counting down of the preparation time, the wearable device may initialize (or calibrate) sensor data output from a sensor module of the wearable device. When the UI screens 910 and 920 are provided, a description of the walking ability measurement scheme may be provided through an audio signal.

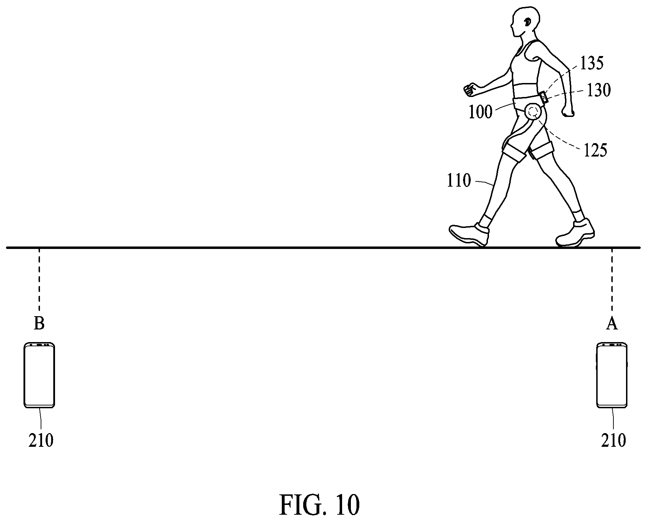

When the counting down of the preparation time is completed, the user wearing the wearable device may start walking along the reference line. The user may walk at a usual gait speed to the end point of the reference line, and the electronic device may obtain sensor data according to walking of the user, using the sensor module during a walking process of the user. For example, referring to FIG. 10, a user 110 may start walking from a start point A of a reference line and walk to an end point B of the reference line according to a guide provided by the electronic device 210. In the walking process, the wearable device 100 obtains sensor data through two or more sensors (including the angle sensor 125 and the IMU 135) included in the sensor module, and transmits the obtained sensor data to the electronic device 210.

Referring back to FIG. 9, in the walking process of the user, a UI screen 930 may be provided from the electronic device. The UI screen 930 may provide a description 932 of the walking ability measurement scheme and information on a period 934 of time that has elapsed from a start of the measurement to a current time.

When the user arrives at the end point and stops walking to be in a standing position, a collection of sensor data for measuring the walking ability of the user may be completed. The wearable device or the electronic device may automatically recognize that the user stops walking based on sensor data obtained from the sensor module of the wearable device. When it is determined that the user is in the standing position when a posture of the user is estimated based on the sensor data, the wearable device or the electronic device may determine that the measurement is completed. When a measurement for an evaluation of the walking ability is completed, the electronic device may provide the user with a UI screen 940 including a description 942 indicating a completion of the measurement and information on a period 944 of time used until the walking is completed.

The electronic device may evaluate a physical ability (e.g., walking ability) of the user by analyzing sensor data obtained up to a point in time at which an end of the walking is recognized. In an embodiment, when the user wears the wearable device and walks a distance of the reference line, the electronic device may automatically estimate gait indices. The electronic device may extract one or more feature points associated with the physical ability of the user from the sensor data, and analyze the physical ability of the user based on the extracted one or more feature points. The electronic device may provide the user with a UI screen 950 indicating physical ability information 952 on the estimated physical ability. In an embodiment, the electronic device may further provide information on an average physical ability of a user group to which an age group of the user belongs and/or information on a previous measurement result as well as information on a measured physical ability of the user.

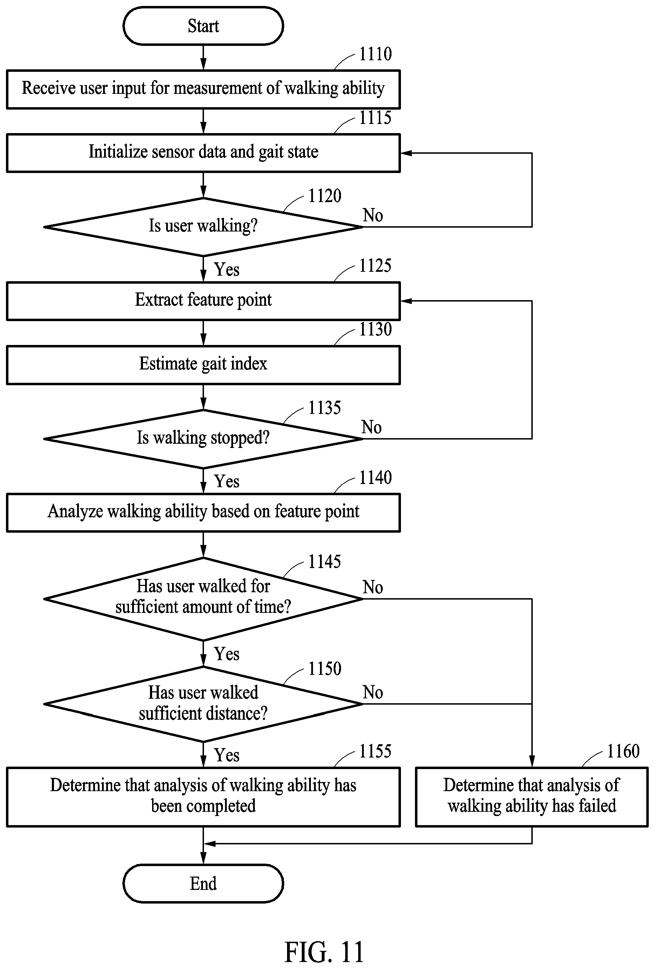

FIG. 11 is a flowchart illustrating a method of measuring a walking ability of a user according to an embodiment.

In operation 1110, the electronic device 210 may receive a user input for a measurement of a walking ability (e.g., a command to perform a walking ability measurement test). The electronic device 210 may request the wearable device 100 to activate a physical ability measurement mode for measuring a walking ability.

In operation 1115, the wearable device 100 may activate the physical ability measurement mode and initialize a gait state of the user and sensor data output from the sensor module. Subsequently, the wearable device 100 may continue to transmit sensor data obtained by the sensor module of the wearable device 100 to the electronic device 210 until the physical ability measurement mode ends.

**In** operation 1120, the electronic device 210 may determine whether the user is walking based on the sensor data received from the wearable device 100. When it is determined that the user is not walking (No in operation 1120), the electronic device 210 may maintain a state in which the sensor data and the gait state are initialized.

When the user wearing the wearable device 100 starts walking according to a guide for the measurement of the walking ability, the electronic device 210 may recognize that the user is walking, based on sensor data. The user may start walking along a reference line of a defined distance. When it is determined that the user is walking (Yes in operation 1120), the electronic device 210 may extract a feature point from the sensor data received from the wearable device 100 in operation 1125. For example, the electronic device 210 may extract a feature point from a signal of a motion value output from an IMU of the wearable device 100. The electronic device 210 may extract a feature point corresponding to a heel strike and a feature point corresponding to a toe-off during a gait motion of the user from the signal of the motion value. In addition, the electronic device 210 may also recognize a gait phase of the user based on a hip joint angle value output from an angle sensor of the wearable device 100.

In operation 1130, the electronic device 210 may estimate a gait index of the user based on the extracted feature points. For example, the electronic device 210 may estimate at least one of a temporal gait index, such as a step time, a swing time, a stance time, a stride time, and a double support time of the user, a spatial gait index, such as a step length, a stride length, and a leg length of the user, a gait fluctuation index, or a gait symmetry index, based on feature points. Values of gait indices may continue to be updated until the walking of the user ends.

In operation 1135, the electronic device 210 may determine whether the user has finished walking and is in a walking-stopped state. When walking to an end point of the reference line is completed, the user may stop walking at the end point, and the electronic device 210 may recognize that the user stops walking based on the sensor data. When the user is not in the walking-stopped state (No in operation 1135), the electronic device 210 may continue to extract feature points from the sensor data and may perform a process of estimating a gait index. The extracted feature points and estimated gait indices may be stored.

When the user is recognized to be in the walking-stopped state (Yes in operation 1135), the electronic device 210 may analyze the walking ability of the user based on the stored feature points in operation 1140.

In operation 1145, the electronic device 210 may determine whether the user has walked for a sufficient amount of time. When it is determined that the user has not walked for the sufficient amount of time (No in operation 1145), the electronic device 210 may determine that an analysis of the walking ability has failed in operation 1160. When it is determined that the user has walked for the sufficient amount of time (Yes in operation 1145), the electronic device 210 may determine whether the user has walked a sufficient distance (e.g., a distance greater than or equal to a distance of the reference line) in operation 1150. When it is determined that the user has not walked the sufficient distance (No in operation 1150), the electronic device 210 may determine that the analysis of the walking ability has failed in operation 1160. When it is determined that the analysis of the walking ability has failed, the electronic device 210 may provide a guide for re-measurement to the user. When it is determined that the user has walked the sufficient distance (Yes in operation 1150), the electronic device 210 may determine that the analysis of the walking ability has been completed in operation 1155. The electronic device 210 may provide the user with walking ability information as a result of the analysis of the walking ability.

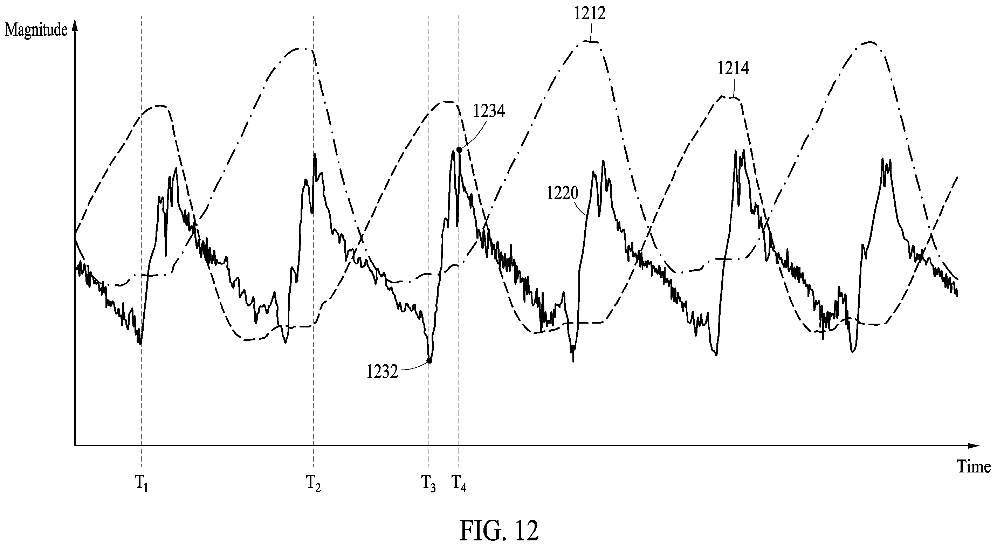

FIG. 12 is a diagram illustrating an example of extracting feature points from sensor data for a measurement of a walking ability according to an embodiment.

Referring to FIG. 12, the electronic device 210 may extract feature points for a calculation of a gait index from sensor data obtained from a sensor module of the wearable device 100. The electronic device 210 may recognize a gait phase of a user based on a hip joint angle value 1212 of a right leg of the user and a hip joint angle value 1214 of a left leg of the user that are measured by an angle sensor of the wearable device 100, and an upper body motion value 1220 of the user measured by an IMU of the wearable device 100, and may extract feature points. The electronic device 210 may recognize, for example, based on the sensor data, that an interval between times T₁ and T₂ corresponds to a left stance in the gait phase, and that an interval between times T₂ and T₃ corresponds to a left swing. For example, a sensor data-based finite-state machine (FSM) scheme may be used for a recognition of a gait phase.

The electronic device 210 may extract feature points from the upper body motion value 1220 in each gait phase. For example, the electronic device 210 may extract a first feature point 1232 corresponding to a heel strike at time T₃ and extract a second feature point 1234 corresponding to a toe-off at time T₄, from the upper body motion value 1220. When the user puts his or her feet on the ground, a back-and-forth motion of an upper body may be stopped, and such an upper body motion may be reflected to the upper body motion value 1220. When the user takes his or her foot off the ground, the upper body may move rapidly forward because a propulsion is required, and such an upper body motion may also be reflected to the upper body motion value 1220. Accordingly, information used to estimate a gait index may be obtained by sensing a point in time at which the above upper body motion rapidly changes in the upper body motion value 1220.

When first feature points 1232 corresponding to the heel strike and second feature points 1234 corresponding to the toe-off are extracted, the electronic device 210 may estimate temporal gait indices. For example, a time between neighboring first feature points 1232 may be determined as a stride time. A time interval between a heel strike of the left leg and a heel strike of the right leg may be determined as a right step time, and a time interval between the heel strike of the right leg and the heel strike of the left leg may be determined as a left step time. A time interval between a toe-off of the right leg and the heel strike of the right leg may be determined as a right swing time, and a time interval between a toe-off of the left leg and the heel strike of the left leg may be determined as a left swing time. A double support time may be determined based on a sum of a time interval between the heel strike of the right leg and the toe-off of the left leg and a time interval between the heel strike of the left leg and the toe-off of the right leg.

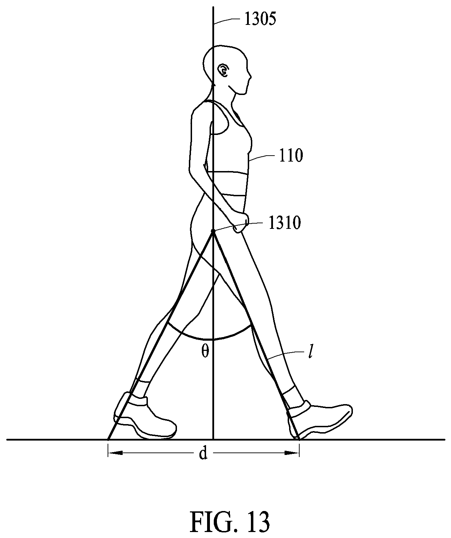

FIG. 13 is a diagram illustrating an example of estimating a spatial gait index of a user according to an embodiment.

Referring to FIG. 13, the electronic device 210 may estimate a spatial gait index, for example, a step length, a stride length, and a leg length of the user 110, based on sensor data (e.g., a hip joint angle value). A length from a position 1310 of a hip joint of the user 110 to each of the right leg and the left leg may be defined by *l*, and a sum of an angle formed between a line 1305 perpendicular to the ground and passing through the position 1310 of the hip joint and a line connecting the position 1310 to a left foot of the user 110 when the left foot contacts the ground, and an angle formed between the line 1305 and a line connecting the position 1310 to a right foot of the user 110 when the right foot contacts the ground may be defined as *θ*.

The electronic device 210 may estimate a stride length *d* based on Equation 1 shown below. [Equation 1] $d = 2 \times l \times sin \frac{θ}{2}$

If the user walks 10 m which is the distance of the reference line, walking angles obtained for each gait cycle are denoted by *θ*₁, *θ*₂, ..., and *θₙ*, the user may estimate a leg length *l* of the user based on Equation 2 shown below. If the leg length *l* of the user is estimated, a gait index (e.g., a stride) may be calculated based on the leg length *l*. [Equation 2] $\begin{matrix}10 = 2 ⋅ l ⋅ \left(sin\left(\frac{{θ}_{1}}{2}\right)+sin\left(\frac{{θ}_{2}}{2}\right)+…+sin\left(\frac{{θ}_{n}}{2}\right)\right) \\ l = \frac{5}{{\sum }_{k = 1}^{n} sin \left(\frac{{θ}_{k}}{2}\right)}\end{matrix}$

In an embodiment, the electronic device 210 may extract a walking interval in which the user 110 stably walks from all walking intervals of the user 110. In an embodiment, the electronic device 210 may extract a walking interval based on sensor data other than sensor data obtained around a start time and an end time of walking. The electronic device 210 may determine a sum of step lengths and a total period of walking time, which are estimated in the extracted walking interval, and may estimate an average gait speed of the user 110 by dividing the sum of the step lengths by the total period of walking time.

FIG. 14 is a flowchart illustrating a method of measuring a physical ability through a TUG test according to an embodiment.

Referring to FIG. 14, the electronic device 210 may receive a user input to perform the TUG test. In response to the user input, the electronic device 210 may request the wearable device 100 to activate a physical ability measurement mode for performing the TUG test. For the TUG test, a chair may be prepared and a predetermined distance from the chair may be marked. In the TUG test, when the TUG test is started in a state in which a user wears the wearable device 100 and sits in the chair, a period of time, in which the user rises from the chair, travels back and forth the predetermined distance and sits in the chair again, may be measured.

Referring to the above measurement process, in operation 1410, the electronic device 210 may count down a preparation time to perform the TUG test, and the wearable device 100 may initialize a hip joint angle value output from an angle sensor during the preparation time. For example, the wearable device 100 may set the hip joint angle value output from the angle sensor during the preparation time to "0". In an embodiment, the electronic device 210 may provide the user with preparations for the TUG test (e.g., a chair, and a tape measure), a test setting scheme, and a guide including a description of a motion to be made by the user wearing the wearable device 100, before the TUG test is started.

When the counting down of the preparation time is completed, the electronic device 210 may notify the user of the start of the TUG test and activate a timer for measuring an amount of time in operation 1420. When the user moves his or her body according to a TUG test scheme, the wearable device 100 may obtain a hip joint angle value using the sensor module and transmit the obtained hip joint angle value to the electronic device 210. In operation 1430, the electronic device 210 may determine whether the hip joint angle value is greater than a first threshold. When the hip joint angle value is greater than the first threshold (Yes in operation 1430), the electronic device 210 may recognize that the user rises from the chair and may change a motion state value of the user to a first state value in operation 1440.

In operation 1450, the electronic device 210 may determine whether the hip joint angle value is less than a second threshold. Here, the second threshold may be different from the first threshold. For example, the second threshold may be less than the first threshold. When the hip joint angle value is less than the second threshold (Yes in operation 1450), the electronic device 210 may recognize that the user sits in the chair again and may change the motion state value of the user to a second state value in operation 1460.

In operation 1470, the electronic device 210 may change the motion state value to a third state value after a predefined period of time (e.g., two seconds) elapses. Here, the first state value, the second state value, and the third state value may be different from each other.

In operation 1480, the electronic device 210 may extract a feature point from the hip joint angle value and evaluate a physical ability of the user according to the TUG test based on the extracted feature point. The electronic device 210 may extract, as a feature point, a hip joint angle value that is a minimum/low value among hip joint angle values measured in an interval in which the motion state value of the user is the second state value. A point in time corresponding to the hip joint angle value that is the minimum/low value may be estimated as a point in time at which the user sits in the chair again.

In an embodiment, for the above TUG test, a hip joint angle value corresponding to a right leg, a hip joint angle value corresponding to a left leg, or an average value of the hip joint angle value corresponding to the right leg and the hip joint angle value corresponding to the left leg may be used. In an embodiment, the electronic device 210 may also perform the TUG test based on a hip joint angle value processed by filtering hip joint angle values.

In an embodiment, when at least one of a case in which the user moves his or her body a lot during the preparation time, a case in which the user is standing during the preparation time, a case in which the user continues to walk a predetermined distance or more instead of traveling back and forth a predetermined distance, a case in which the user does not sit in the chair again for a defined period of time after rising from the chair, or a case in which a number of walking steps of the user is a reference number of steps or less and the user sits in the chair again, is detected, the electronic device 210 may output an error message to the user and propose to re-perform the TUG test.

FIG. 15 is a diagram illustrating an example of extracting a feature point of sensor data to measure a physical ability in a TUG test according to an embodiment.

FIG. 15 illustrates an example of a hip joint angle value 1510 corresponding to a right leg and a hip joint angle value 1520 corresponding to a left leg obtained during the TUG test, over time. When the TUG test is started, the electronic device 210 may count down a preparation time. During a period of time (e.g., between times T₁ and T₂) counted down, the wearable device 100 may initialize (or calibrate) a hip joint angle value output from the angle sensor. In an embodiment, the wearable device 100 may set an initial value of the hip joint angle value as an average value of hip joint angle values obtained during the preparation time.

When the counting down is completed, the TUG test may be started at the time T₂, and a user may rise from a chair. Accordingly, a hip joint angle value may change. When a time T₃, at which at least one of the hip joint angle value 1510 corresponding to the right leg and the hip joint angle value 1520 corresponding to the left leg becomes greater than a first threshold, is recognized, the electronic device 210 may recognize that the user rises from the chair and may change a motion state value to a first state value. The user may rise from the chair and travel back and forth a predetermined distance, and accordingly the hip joint angle value 1510 corresponding to the right leg and the hip joint angle value 1520 corresponding to the left leg may be changed according to a gait phase. If the user reaches the chair and sits in the chair again, the hip joint angle value 1510 corresponding to the right leg and the hip joint angle value 1520 corresponding to the left leg may decrease. When a time T₄, at which at least one of the hip joint angle value 1510 corresponding to the right leg and the hip joint angle value 1520 corresponding to the left leg is less than a second threshold, is recognized, the electronic device 210 may recognize that the user sits in the chair and may change the motion state value to a second state value. The electronic device 210 may change the motion state value to a third state value at a time T₆ that elapses by a predefined period of time from the time T₄.

The electronic device 210 may recognize a time T₅ corresponding to a hip joint angle value that is a minimum/low value in an interval (between the times T₄ and T₆) in which the motion state value is the second state value, and may extract the hip joint angle value at the time T₅ as a feature point. The electronic device 210 may estimate a physical ability of the user based on a period of time from the time T₂ at which the TUG test is started to the time T₅ at which the feature point is extracted. If the period of time from the time T₂ to the time T₅ decreases, a walking ability and a balance ability of the user may be evaluated to be excellent. "Based on" as used herein covers based at least on.

FIG. 16 is a flowchart illustrating a method of measuring a physical ability through a 5×STS test according to an embodiment.

Referring to FIG. 16, the electronic device 210 may receive a user input to perform the 5×STS test. In response to the user input, the electronic device 210 may request the wearable device 100 to activate a physical ability measurement mode for performing the 5×STS test. For the 5×STS test, a chair in which a user is to sit may be prepared. In the 5×STS test, a period of time, in which the user wears the wearable device 100 and repeatedly performs a motion of rising from the chair and sitting in the chair a set number of times, may be measured.

Referring to the above measurement process, in operation 1610, the electronic device 210 may count down a preparation time for the 5×STS test, and the wearable device 100 may initialize a hip joint angle value output from the angle sensor during the preparation time. In an embodiment, the electronic device 210 may provide the user with preparations (e.g., a chair) for the 5×STS test, a test setting scheme, and a guide including a description of a motion to be made by the user wearing the wearable device 100, before the 5×STS test is started.

When the counting down of the preparation time is completed, the electronic device 210 may notify the user of the start of the 5×STS test and activate a timer for measuring an amount of time in operation 1620. When the user moves his or her body according to a 5×STS test scheme, the wearable device 100 may obtain a hip joint angle value using the sensor module (comprising at least one sensor) and transmit the obtained hip joint angle value to the electronic device 210. In operation 1630, the electronic device 210 may determine whether the hip joint angle value is greater than a first threshold. When the hip joint angle value is greater than the first threshold (Yes in operation 1630), the electronic device 210 may recognize that the user rises from the chair, may change a motion state value of the user to a first state value, and may increase a number of motions by "1" in operation 1640. For example, if it is first recognized that the hip joint angle value is greater than the first threshold, the number of motions may be increased from an initial value of "0" to "1." The first state value may indicate that the user is standing up.

Subsequently, in operation 1650, the electronic device 210 may determine whether the hip joint angle value is less than a second threshold. Here, the second threshold may be different from the first threshold. For example, the second threshold may be less than the first threshold. When the hip joint angle value is less than the second threshold (Yes in operation 1650), the electronic device 210 may recognize that the user sits in the chair again and may change the motion state value of the user to a second state value in operation 1670. Here, the second state value may indicate that the user sits in the chair.

In operation 1680, the electronic device 210 may determine whether the number of motions reaches a reference number of times (e.g., five times). The number of motions may correspond to a number of times the user rises from the chair and sits in the chair. When the number of motions does not reach the reference number of times (No in operation 1680), the electronic device 210 may perform the above process again from operation 1630.

When the number of motions reaches the reference number of times (Yes in operation 1680), the electronic device 210 may extract feature points from hip joint angle values recorded up to a current time and evaluate a physical ability of the user according to the 5×STS test based on the extracted feature points in operation 1690. The electronic device 210 may extract, as a feature point, a hip joint angle value that is a maximal or large value in a time interval corresponding to the first state value appearing immediately before when the number of motions reaches the reference number of times. The electronic device 210 may estimate the physical ability of the user based on a period of time from the start of the 5×STS test to an extraction of a corresponding feature point. If the period of time decreases, a muscle strength of a leg of the user may be evaluated to be excellent.

In an embodiment, for the above 5×STS test, a hip joint angle value corresponding to a right leg, a hip joint angle value corresponding to a left leg, or an average value of the hip joint angle value corresponding to the right leg and the hip joint angle value corresponding to the left leg may be used. In an embodiment, the electronic device 210 may also perform the 5×STS test based on a hip joint angle value processed by filtering hip joint angle values.

In an embodiment, when at least one of a case in which the user moves his or her body a lot during the preparation time, a case in which the user is standing during the preparation time, or a case in which the user moves less than a reference number of times and does not rise from the chair for a defined period of time, is detected, the electronic device 210 may output an error message to the user and propose to re-perform the 5×STS test.

FIG. 17 is a diagram illustrating an example of extracting a feature point of sensor data to measure a physical ability in a 5×STS test according to an embodiment.

FIG. 17 illustrates an example of a hip joint angle value 1710 corresponding to a right leg obtained during the 5×STS test, over time. According to an embodiment, for the 5×STS test, a hip joint angle value corresponding to a left leg, or an average value of a hip joint angle value corresponding to a right leg and the hip joint angle value corresponding to the left leg may be used.

If the 5×STS test is started, the electronic device 210 may count down a preparation time. During a period of time (between times T₁ and T₂) counted down, the wearable device 100 may initialize (or calibrate) a hip joint angle value output from the angle sensor. In an embodiment, the wearable device 100 may set the hip joint angle value as an initial value based on an average value of hip joint angle values obtained during the preparation time.

When the counting down is completed, the 5×STS test may be started at the time T₂, and a user may rise from a chair. Accordingly, a hip joint angle value may change. When a time T₃, at which the hip joint angle value 1710 becomes greater than a first threshold, is recognized, the electronic device 210 may recognize that the user rises from the chair, may change a motion state value to a first state value, and may increase a number of motions from "0" to "1".

While the user is sitting in the chair again, the measured hip joint angle value 1710 may gradually decrease. When a time T₄, at which the hip joint angle value 1710 is less than a second threshold, is recognized, the electronic device 210 may recognize that the user sits in the chair and may change the motion state value to a second state value.

Subsequently, a process in which the user rises from the chair again and sit in the chair may be repeatedly performed. In the process, times T₅, T₇, T₉, and T₁₁, at which it is recognized that the user rises from the chair, and times T₆, T₈, T₁₀, and T₁₃, at which it is recognized that the user sits in the chair again may be detected. In a process in which the user repeatedly stands up and sits down, the number of motions may gradually increase. At a predetermined time, the number of motions may reach a reference number of times. When the number of motions reaches the reference number of times, the electronic device 210 may extract, as a feature value, a hip joint angle value that is a maximal/high value in a time interval (between times T₁₁ and T₁₃) corresponding to the first state value appearing immediately before when the number of motions reaches the reference number of times, in the hip joint angle value 1710. The extracted feature point may correspond to a hip joint angle value extracted at a time T₁₂. The electronic device 210 may estimate a physical ability of the user based on a period of time from the time T₂ at which the 5×STS test is started to the time T₁₂ at which the feature point is extracted.

FIG. 18 is a flowchart illustrating a method of measuring a physical ability through an SPPB test according to an embodiment.

Referring to FIG. 18, the electronic device 210 may receive a user input to perform the SPPB test. In response to the user input, the electronic device 210 may request the wearable device 100 to activate a physical ability measurement mode for performing the SPPB test. In the SPPB test, a user may wear the wearable device 100 and stand in a predetermined leg posture, and a degree of a motion of the user may be measured during a test time.

Referring to the above measurement process, in operation 1810, the electronic device 210 may count down a preparation time for the SPPB test, and the wearable device 100 may initialize sensor data output from a sensor module during the preparation time. For example, the wearable device 100 may initialize a hip joint angle value output from an angle sensor and an upper body motion value output from an IMU. In an embodiment, the electronic device 210 may provide the user with a test setting scheme for the SPPB test, and a guide including a description of a motion to be made by the user wearing the wearable device 100, before the SPPB test is started.

When the counting down of the preparation time is completed, the electronic device 210 may notify the user of the start of the SPPB test and activate a timer for measuring an amount of time in operation 1820.

In operation 1830, the electronic device 210 may record a motion of a leg for a preset period of time. In operation 1840, the electronic device 210 may evaluate a physical ability of the user based on the motion of the leg. The electronic device 210 may estimate a degree of the motion of the leg based on the hip joint angle value and may estimate a degree of a motion of an upper body of the user based on the upper body motion value. When a hip joint angle value measured for one time interval (e.g., ten seconds) is greater than or equal to a first threshold, the electronic device 210 may determine that the leg of the user moves. When an upper body motion value measured for one time interval is greater than or equal to a second threshold, the electronic device 210 may determine that the upper body of the user moves.

In an embodiment, the electronic device 210 may estimate the physical ability (e.g., a balance ability) of the user, based on a posture of a user on which the SPPB test is to be performed, the upper body motion value measured for one time interval, and a time at which it is determined that the leg moves. The electronic device 210 may assign different scores for each of the posture of the user, the upper body motion value, and a time in which the leg moves, and may determine a final score for the SPPB test of the user based on scores of respective evaluation factors. For example, when the degree of the motion of the upper body and/or the degree of the motion of the legs is large in the SPPB test, the electronic device 210 may assign a relatively low score. In addition, when the time in which the leg of the user moves decreases, a score assigned by the electronic device 210 may decrease.

In an embodiment, the electronic device 210 may calculate a score associated with an evaluation of the physical ability of the user, based on an evaluation criterion for evaluating the physical ability of the user through the SPPB test. For example, the electronic device 210 may determine a score for the user based on evaluation criteria shown in Table 1 below. The degree of the motion of the upper body may be determined based on, for example, an upper body motion value and/or a hip joint angle value.

**[Table 1]**

| | | Score assignment criteria according to postures | | |
|---|---|---|---|---|
| Leg motion sensing time | Degree of motion of upper body | Side-by-side stance | Semi-tandem stance | Tandem stance |
| Between 0 and 3 seconds | (irrelevant) | 0 | 0 | 0 |
| Between 3 and 10 seconds | | 0 | 0 | 1 |
| After at least 10 seconds | Large | 0 | 0 | 0 |
| | Medium | 1 | 1 | 1 |
| | Small | 1 | 1 | 2 |

In an embodiment, when a sitting posture of the user is detected during the SPPB test, the electronic device 210 may output an error message to the user and propose to re-perform the SPPB test.

According to various embodiments of the present disclosure, a user may easily measure a physical ability by himself/herself using the wearable device 100 and the electronic device 210 even though a separate measuring person and separate measurement equipment are absent. Through the embodiments, a walking ability, a muscle strength, a potential fall, a balance ability, and the like of the user may be relatively accurately evaluated.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things, unless the relevant context clearly indicates otherwise. As used herein, "A or B," "at least one of A and B," "at least one of A or B," "A, B or C," "at least one of A, B and C," and "at least one of A, B, or C," may include any one of the items listed together in the corresponding one of the phrases, or all possible combinations thereof. Terms such as "1st" and "2nd," or "first" and "second" may be used to simply distinguish a corresponding component from other components, and do not limit the components in other aspects (e.g., importance or order). It is to be understood that if an element (e.g., a first element) is referred to, with or without the term "operatively" or "communicatively," as "coupled with," "coupled to," "connected with," or "connected to" another element (e.g., a second element), the element may be coupled with the other element directly (e.g., by wire), wirelessly, or via at least a third element.

As used in connection with various embodiments of the disclosure, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an embodiment, the module may be implemented in a form of an application-specific integrated circuit (ASIC). Thus, each "module" herein may comprise circuitry.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording mediums. Embodiments as set forth herein may be implemented as software including one or more instructions that are stored in a storage medium (e.g., the memory 514) that is readable by a machine. For example, a processor of the machine may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments may be included and provided in a computer program product. The computer program product may be traded as a product between a seller and a buyer. The computer program product may be distributed in the form of a machine-readable storage medium (e.g., compact disc read-only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as memory of the manufacturer's server, a server of the application store, or a relay server.

According to embodiments, each component (e.g., a module or a program) of the above-described components may include a single entity or multiple entities, and some of the multiple entities may be separately disposed in different components. According to embodiments, one or more of the above-described components may be omitted, or one or more other components may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to embodiments, operations performed by the module, the program, or another component may be carried out sequentially, in parallel, repeatedly, or heuristically, or one or more of the operations may be executed in a different order or omitted, or one or more other operations may be added.

While the disclosure has been illustrated and described with reference to various embodiments, it will be understood that the various embodiments are intended to be illustrative, not limiting. It will also be understood that any of the embodiment(s) described herein may be used in conjunction with any other embodiment(s) described herein.

## Claims

1. A wearable device (100), comprising:
a leg support frame (50,55) configured to support a leg of a user when the wearable device (100) is worn on a body of the user;
a first waist support frame (25) and a second waist support frame (20) configured to support a waist of the user when the wearable device (100) is worn on the body of the user;
a sensor module (520), comprising at least two sensors, configured to obtain sensor data including motion information of the user in a physical ability measurement mode ;
a communication module (516), comprising communication circuitry, configured to transmit the sensor data to an electronic device (210) and receive at least a control signal from the electronic device (210); and
at least one processor (512) configured to control the communication module (516) and the sensor module (520),
wherein the sensor module (520) is configured to obtain first sensor data including motion information of the user to be measured by an inertial sensor and second sensor data including a hip joint angle of the user to be measured by an angle sensor,
wherein the second sensor data comprises information on the hip joint angle of the user measured by an angle sensor of the wearable device (100) during a walking process of the user performed from a start point to an end point, and
wherein the at least one processor (512) is configured to control the communication module (516) to transmit the first sensor data and the second sensor data to the electronic device (210) in the physical ability measurement mode, wherein the at least one processor (512) is further configured to, when the control signal, for a measurement of a target physical ability of the user, is received from the electronic device (210):
activate the physical ability measurement mode in response to the control signal; and
control the sensor module (520) to obtain the sensor data in the physical ability measurement mode, and wherein the at least one processor (512) is configured, when the physical ability measurement mode is activated, to initialize the sensor data and control a recording of the sensor data to be started when a predetermined period of time for measuring the target physical ability elapses.

2. A system comprising the wearable device (100) of claim 1 and an electronic device (210), the electronic device (210) comprising:
a communication module (730), comprising communication circuitry, configured to receive sensor data from a wearable device (100) worn on a waist and a leg of a user and to send at least a control signal to the wearable device (100);
at least one processor (710) configured to determine physical ability information of the user based on the sensor data; and
a display module (740), comprising a display (212), configured to output the physical ability information via a user interface screen,
wherein the at least one processor (710) is configured to:
recognize that the user ends walking based on the received sensor data, and when it is recognized that the user ends walking, complete a collection of sensor data for measuring a walking ability of the user; and
determine information on the walking ability of the user based on sensor data obtained up to a point in time at which an end of the walking is recognized,
wherein the at least one processor is further configured to:
calculate a temporal gait index based on first sensor data including motion information of the user received from the wearable device (100);
calculate a spatial gait index based on second sensor data regarding a hip joint angle of the user received from the wearable device (100); and
calculate the physical ability information based on the temporal gait index and the spatial gait index,
wherein the second sensor data comprises information on the hip joint angle of the user measured by an angle sensor of the wearable device (100)during a walking process of the user performed from a start point to an end point.

3. The system of claim 2, wherein the at least one processor (710) of the electronic device (210) is configured to calculate the temporal gait index based on a time interval between first feature points (1232) temporally adjacent to each other, a time interval between second feature points (1234) temporally adjacent to each other, and a time interval between a first feature point (1232) and a second feature point (1234) temporally adjacent to the first feature point (1232),
wherein the temporal gait index comprises at least one of: a step time, a swing time, a stance time, a stride time, or a double support time of the user.

4. The system of one of claims 2 to 3, wherein the at least one processor (710) of the electronic device (210) is configured to calculate the spatial gait index based on a hip joint angle value at a point in time corresponding to a heel strike from the hip joint angle value of the user measured by the angle sensor of the wearable device (100),
wherein the spatial gait index comprises at least one of: a step length, a stride length, or a leg length of the user.

5. The system of one of claims 2 to 4, wherein the at least one processor (710) of the electronic device (210) is configured to calculate at least one of a gait speed, a gait fluctuation index, or a gait symmetry index of the user, based on the temporal gait index and the spatial gait index.

6. The system of one of claims 2 to 5, wherein the at least one processor (710) of the electronic device (210) is configured to provide a user interface to guide a measurement of a target physical ability of the user to the user, via the display module (740).

7. The system of claim 6, wherein the at least one processor (710) of the electronic device (210) is configured to, based on a user input for the measurement of the target physical ability of the user being received, control the communication module (730) to transmit a control signal for the measurement of the target physical ability to the wearable device (100) in response to the user input.

8. The system of one of claims 2 to 7, wherein the at least one processor (710) of the electronic device (210) is configured to, when a physical ability measurement mode for measuring a target physical ability is a timed up and go, TUG, test,
when a hip joint angle value included in the sensor data is greater than or equal to a first threshold, determine that the user rises from a chair;
when the hip joint angle value is less than or equal to a second threshold, determine that the user sits in the chair;
extract, as a feature point, a hip joint angle value corresponding to a minimum value among hip joint angle values measured during a predefined period of time that elapses from a point in time at which it is determined that the user sits in the chair; and
estimate the physical ability information of the user based on a period of time from a start of the TUG test until the hip joint angle value corresponding to the minimum value is extracted.

9. The system of one of claims 2 to 7, wherein the at least one processor (710) of the electronic device (210) is configured to, when a physical ability measurement mode for measuring a target physical ability is a five times sit to stand, 5xSTS, test:
when a hip joint angle value included in the sensor data is greater than or equal to a first threshold, determine that the user rises from a chair and increase a number of times the user rises from the chair;
when the hip joint angle value is less than or equal to a second threshold, determine that the user sits in the chair;
when the number of times the user rises from the chair reaches a reference number of times, extract a hip joint angle value corresponding to a maximum value appearing immediately before the number of times the user rises from the chair reaches the reference number of times, as a feature point; and
calculate physical ability information of the user based on a period of time from a start of the 5×STS test until the hip joint angle value corresponding to the maximum value is extracted.

10. The system of one of claims 2 to 7, wherein the at least one processor (710) of the electronic device (210) is configured to, when a physical ability measurement mode for measuring a target physical ability is a short physical performance battery, SPPB, test:
when a hip joint angle value measured for one time interval is greater than or equal to a first threshold, determine that a leg of the user moves; and
calculate the physical ability information of the user based on a posture of the user on which the SPPB test is to be performed, an upper body motion value measured during the one time interval, and a time at which it is determined that the leg moves.

11. A method of operating a wearable device (100), the wearable device (100) comprising a leg support frame configured to support a leg of a user, a first waist support frame (25) and a second waist support frame (20) configured to support a waist of the user when the wearable device (100) is worn on a body of the user, a sensor module (520), comprising at least two sensors, configured to obtain sensor data including motion information of the user in a physical ability measurement mode, and a communication module (516), comprising communication circuitry, configured to transmit the sensor data to an electronic device (210) and receive at least a control signal from the electronic device (210), the method comprising:
receiving a control signal for a measurement of a target physical ability of the user from an electronic device (210);
activating a physical ability measurement mode in response to the control signal, when the physical ability measurement mode is activated, initializing
sensor data and controlling a recording of the sensor data to be started when a predetermined period of time for measuring the target physical ability elapses, and obtaining first sensor data including motion information of the user measured by an inertial sensor of the wearable device (100) and second sensor data including a hip joint angle of the user measured by an angle sensor of the wearable device (100); and transmitting the first sensor data and the second sensor data to the electronic device (210), so that the electronic device (210) calculates a temporal gait index based on the first sensor data, and calculates a spatial gait index based on the second sensor data,
wherein the second sensor data comprises information on the hip joint angle of the user measured by an angle sensor of the wearable device (100) during a walking process of the user performed from a start point to an end point.

12. A method, according to claim 11, of operating the system of claim 2,
wherein the method further comprises, by the electronic device (210):
receiving a user input for a measurement of a target physical ability of a user;
transmitting the control signal for the measurement of the target physical ability to the wearable device (100) in response to the user input;
receiving the first sensor data and the second sensor data from the wearable device (100);
determining physical ability information regarding the target physical ability of the user based on the first sensor data and the second sensor data,
wherein the determining comprises:
when the electronic device (210) recognizes that the user ends walking based on the received sensor data, completing a collection of sensor data for measuring a walking ability of the user;
determining information on the walking ability of the user based on sensor data obtained up to a point in time at which the end of walking is recognized;
wherein the determining of the information on the walking ability comprises calculating a temporal gait index based on the first sensor data and a spatial gait index based on the second sensor data, and calculating the physical ability information based on the temporal gait index and the spatial gait index; and
providing, via a user interface screen, the physical ability information to the user.

## Patentansprüche

1. Wearable-Vorrichtung (100), umfassend:
einen Beinstützrahmen (50, 55), der dazu konfiguriert ist, ein Bein eines Benutzers abzustützen, wenn die Wearable-Vorrichtung (100) am Körper des Benutzers getragen wird;
einen ersten Taillenstützrahmen (25) und einen zweiten Taillenstützrahmen (20), die dazu konfiguriert sind, die Taille des Benutzers abzustützen, wenn die Wearable-Vorrichtung (100) am Körper des Benutzers getragen wird;
ein Sensormodul (520), das mindestens zwei Sensoren umfasst und dazu konfiguriert ist, in einem Messmodus für physikalische Fähigkeiten Sensordaten einschließlich Bewegungsinformationen des Benutzers zu erhalten;
ein Kommunikationsmodul (516), das eine Kommunikationsschaltung umfasst und dazu konfiguriert ist, die Sensordaten an eine elektronische Vorrichtung (210) zu übertragen und mindestens ein Steuersignal von der elektronischen Vorrichtung (210) zu empfangen; und
mindestens einen Prozessor (512), der dazu konfiguriert ist, das Kommunikationsmodul (516) und das Sensormodul (520) zu steuern,
wobei das Sensormodul (520) dazu konfiguriert ist, erste Sensordaten, die von einem Trägheitssensor zu messende Bewegungsinformationen des Benutzers umfassen, und zweite Sensordaten, die einen von einem Winkelsensor zu messenden Hüftgelenkswinkel des Benutzers umfassen, zu erhalten,
wobei die zweiten Sensordaten Informationen über den Hüftgelenkswinkel des Benutzers umfassen, der von einem Winkelsensor der Wearable-Vorrichtung (100) während eines von einem Startpunkt zu einem Endpunkt durchgeführten Gehvorgangs des Benutzers gemessen wird, und
wobei der mindestens eine Prozessor (512) dazu konfiguriert ist, das Kommunikationsmodul (516) zu steuern, um die ersten Sensordaten und die zweiten Sensordaten im Messmodus für physikalische Fähigkeiten an die elektronische Vorrichtung (210) zu übertragen werden, wobei der mindestens eine Prozessor (512) ferner so konfiguriert ist, dass er, wenn das Steuersignal zur Messung einer angestrebten physikalischen Fähigkeit des Benutzers von der elektronischen Vorrichtung (210) empfangen wird:
den Messmodus für physikalische Fähigkeiten als Reaktion auf das Steuersignal aktiviert; und
das Sensormodul (520) steuert, um die Sensordaten im Messmodus für physikalische Fähigkeiten zu erhalten, und wobei der mindestens eine Prozessor (512) so konfiguriert ist, dass er, wenn der Messmodus für physikalische Fähigkeiten aktiviert ist, die Sensordaten initialisiert und eine Aufzeichnung der Sensordaten so steuert, dass sie beginnt, wenn eine vorbestimmte Zeitspanne zur Messung der angestrebten physikalischen Fähigkeit abgelaufen ist.

2. System, umfassend die Wearable-Vorrichtung (100) nach Anspruch 1 und eine elektronische Vorrichtung (210), wobei die elektronische Vorrichtung (210) Folgendes umfasst:
ein Kommunikationsmodul (730), das eine Kommunikationsschaltung umfasst und dazu konfiguriert ist, Sensordaten von einer an der Taille und einem Bein eines Benutzers getragenen Wearable-Vorrichtung (100) zu empfangen und mindestens ein Steuersignal an die Wearable-Vorrichtung (100) zu senden;
mindestens einen Prozessor (710), der dazu konfiguriert ist, auf der Grundlage der Sensordaten physikalische Fähigkeitsinformationen des Benutzers zu bestimmen; und
ein Anzeigemodul (740), das eine Anzeige (212) umfasst und dazu konfiguriert ist, die physikalischen Fähigkeitsinformationen über einen Benutzeroberflächenbildschirm auszugeben,
wobei der mindestens eine Prozessor (710) dazu konfiguriert ist:
auf der Grundlage der empfangenen Sensordaten zu erkennen, dass der Benutzer das Gehen beendet, und, wenn erkannt wird, dass der Benutzer das Gehen beendet, eine Sammlung von Sensordaten zur Messung einer Gehfähigkeit des Benutzers abzuschließen; und
Informationen über die Gehfähigkeit des Benutzers auf der Grundlage von Sensordaten zu bestimmen, die bis zu einem Zeitpunkt erhalten werden, zu dem ein Ende des Gehens erkannt wird,
wobei der mindestens eine Prozessor ferner dazu konfiguriert ist:
einen zeitlichen Gangindex auf der Grundlage erster Sensordaten zu berechnen, die von der Wearable-Vorrichtung (100) empfangene Bewegungsinformationen des Benutzers umfassen;
einen räumlichen Gangindex auf der Grundlage zweiter Sensordaten bezüglich eines von der Wearable-Vorrichtung (100) empfangenen Hüftgelenkwinkels des Benutzers zu berechnen; und
die physikalischen Fähigkeitsinformationen auf der Grundlage des zeitlichen Gangindex und des räumlichen Gangindex zu berechnen,
wobei die zweiten Sensordaten Informationen über den Hüftgelenkswinkel des Benutzers umfassen, der von einem Winkelsensor der Wearable-Vorrichtung (100) während eines von einem Startpunkt zu einem Endpunkt durchgeführten Gehvorgangs des Benutzers gemessen wird.

3. System nach Anspruch 2, wobei der mindestens eine Prozessor (710) der elektronischen Vorrichtung (210) dazu konfiguriert ist, den zeitlichen Gangindex auf der Grundlage eines Zeitintervalls zwischen ersten, zeitlich benachbarten Merkmalspunkten (1232),
eines Zeitintervalls zwischen zweiten, zeitlich benachbarten Merkmalspunkten (1234)
und eines Zeitintervalls zwischen einem ersten Merkmalspunkt (1232) und einem zweiten Merkmalspunkt (1234), der zeitlich an den ersten Merkmalspunkt (1232) angrenzt, zu berechnen,
wobei der zeitliche Gangindex mindestens eines von Folgendem umfasst: eine Schrittzeit, eine Schwungzeit, eine Standzeit, eine Schrittlänge und eine Doppelstützzeit des Benutzers.

4. System nach einem der Ansprüche 2 bis 3, wobei der mindestens eine Prozessor (710) der elektronischen Vorrichtung (210) dazu konfiguriert ist, den räumlichen Gangindex auf der Grundlage eines Hüftgelenkwinkelwerts zu einem Zeitpunkt, der einem Fersenauftritt entspricht, aus dem vom Winkelsensor der Wearable-Vorrichtung (100) gemessenen Hüftgelenkwinkelwert des Benutzers zu berechnen,
wobei der räumliche Gangindex mindestens eines von Folgendem umfasst: eine Schrittlänge, eine Schrittweite und eine Beinlänge des Benutzers.

5. System nach einem der Ansprüche 2 bis 4, wobei der mindestens eine Prozessor (710) der elektronischen Vorrichtung (210) dazu konfiguriert ist, auf der Grundlage des zeitlichen Gangindex und des räumlichen Gangindex mindestens eines von einer Ganggeschwindigkeit, einem Gangschwankungsindex und einem Gangsymmetrieindex des Benutzers zu berechnen.

6. System nach einem der Ansprüche 2 bis 5, wobei der mindestens eine Prozessor (710) der elektronischen Vorrichtung (210) dazu konfiguriert ist, dem Benutzer über das Anzeigemodul (740) eine Benutzeroberfläche bereitzustellen, um eine Messung einer angestrebten physikalischen Fähigkeit des Benutzers zu leiten.

7. System nach Anspruch 6, wobei der mindestens eine Prozessor (710) der elektronischen Vorrichtung (210) dazu konfiguriert ist, auf der Grundlage einer empfangenen Benutzereingabe zur Messung der angestrebten physikalischen Fähigkeit des Benutzers das Kommunikationsmodul (730) zu steuern, um als Reaktion auf die Benutzereingabe ein Steuersignal für die Messung der angestrebten physikalischen Fähigkeit an die Wearable-Vorrichtung (100) zu übertragen.

8. System nach einem der Ansprüche 2 bis 7, wobei der mindestens eine Prozessor (710) der elektronischen Vorrichtung (210) so konfiguriert ist, dass er, wenn es sich bei einem Messmodus für physikalische Fähigkeiten zur Messung einer angestrebten physikalischen Fähigkeit um einen Timed Up and Go-, TUG, -Test handelt,
wenn ein in den Sensordaten enthaltener Hüftgelenkwinkelwert größer oder gleich einem ersten Schwellenwert ist, feststellt, dass der Benutzer von einem Stuhl aufsteht;
wenn der Hüftgelenkwinkelwert kleiner oder gleich einem zweiten Schwellenwert ist, feststellt, dass der Benutzer auf dem Stuhl sitzt;
als Merkmalspunkt einen Hüftgelenkwinkelwert extrahiert, der einem Minimalwert unter den während einer vordefinierten Zeitspanne gemessenen Hüftgelenkwinkelwerten entspricht, die ab einem Zeitpunkt vergeht, zu dem festgestellt wird, dass der Benutzer auf dem Stuhl sitzt; und
die physikalischen Fähigkeitsinformationen des Benutzers auf der Grundlage einer Zeitspanne vom Beginn des TUG-Tests bis zur Extraktion des dem Minimalwert entsprechenden Hüftgelenkwinkelwerts schätzt.

9. System nach einem der Ansprüche 2 bis 7, wobei der mindestens eine Prozessor (710) der elektronischen Vorrichtung (210) so konfiguriert ist, dass er, wenn es sich bei einem Messmodus für physikalische Fähigkeiten zur Messung einer angestrebten physikalischen Fähigkeit um einen Five Times Sit to Stand-, 5xSTS, -Test handelt:
wenn ein in den Sensordaten enthaltener Hüftgelenkwinkelwert größer oder gleich einem ersten Schwellenwert ist, feststellt, dass der Benutzer von einem Stuhl aufsteht, und die Anzahl der Male erhöht, in denen der Benutzer vom Stuhl aufsteht;
wenn der Hüftgelenkwinkelwert kleiner oder gleich einem zweiten Schwellenwert ist, feststellt, dass der Benutzer auf dem Stuhl sitzt;
wenn die Anzahl der Male, in denen der Benutzer vom Stuhl aufsteht, eine Referenzanzahl von Malen erreicht, als Merkmalspunkt einen Hüftgelenkwinkelwert extrahiert, der einem Maximalwert entspricht, der unmittelbar vor dem Erreichen der Referenzanzahl von Malen durch die Anzahl der Male, in denen der Benutzer vom Stuhl aufsteht, auftritt; und
die physikalischen Fähigkeitsinformationen des Benutzers auf der Grundlage einer Zeitspanne vom Beginn des 5xSTS-Tests bis zur Extraktion des dem Maximalwert entsprechenden Hüftgelenkwinkelwerts berechnet.

10. System nach einem der Ansprüche 2 bis 7, wobei der mindestens eine Prozessor (710) der elektronischen Vorrichtung (210) so konfiguriert ist, dass er, wenn es sich bei einem Messmodus für physikalische Fähigkeiten zur Messung einer angestrebten physikalischen Fähigkeit um einen Short Physical Performance Battery-, SPPB, -Test handelt:
wenn ein für ein Zeitintervall gemessener Hüftgelenkwinkelwert größer oder gleich einem ersten Schwellenwert ist, feststellt, dass sich ein Bein des Benutzers bewegt; und
die physikalischen Fähigkeitsinformationen des Benutzers auf der Grundlage einer Körperhaltung des Benutzers, in der der SPPB-Test durchgeführt werden soll, eines während des einen Zeitintervalls gemessenen Oberkörperbewegungswerts und eines Zeitpunkts, zu dem festgestellt wird, dass sich das Bein bewegt, berechnet.

11. Verfahren zum Betreiben einer Wearable-Vorrichtung (100), wobei die Wearable-Vorrichtung (100) einen Beinstützrahmen, der zum Abstützen eines Beins eines Benutzers konfiguriert ist, einen ersten Taillenstützrahmen (25) und einen zweiten Taillenstützrahmen (20), die zum Abstützen der Taille des Benutzers konfiguriert sind, wenn die Wearable-Vorrichtung (100) am Körper des Benutzers getragen wird, ein Sensormodul (520), das mindestens zwei Sensoren umfasst und dazu konfiguriert ist, in einem Messmodus für physikalische Fähigkeiten Sensordaten einschließlich Bewegungsinformationen des Benutzers zu erhalten, und ein Kommunikationsmodul (516) umfasst, das eine Kommunikationsschaltung umfasst und dazu konfiguriert ist, die Sensordaten an eine elektronische Vorrichtung (210) zu übertragen und mindestens ein Steuersignal von der elektronischen Vorrichtung (210) zu empfangen, wobei das Verfahren Folgendes umfasst:
Empfangen eines Steuersignals für eine Messung einer angestrebten physikalische Fähigkeit des Benutzers von einer elektronischen Vorrichtung (210);
Aktivieren eines Messmodus für physikalische Fähigkeiten als Reaktion auf das Steuersignal, wenn der Messmodus für physikalische Fähigkeiten aktiviert ist, Initialisieren von Sensordaten und Steuern einer Aufzeichnung der Sensordaten, die gestartet werden soll, wenn eine vorbestimmte Zeitspanne für die Messung der angestrebten physikalische Fähigkeit abgelaufen ist, und Erhalten erster Sensordaten, die von einem Trägheitssensor der Wearable-Vorrichtung (100) gemessene Bewegungsinformationen des Benutzers umfassen, und zweiter Sensordaten, die einen von einem Winkelsensor der Wearable-Vorrichtung (100) gemessenen Hüftgelenkswinkel des Benutzers umfassen; und
Übertragen der ersten Sensordaten und der zweiten Sensordaten an die elektronische Vorrichtung (210), so dass die elektronische Vorrichtung (210) auf der Grundlage der ersten Sensordaten einen zeitlichen Gangindex und auf der Grundlage der zweiten Sensordaten einen räumlichen Gangindex berechnet,
wobei die zweiten Sensordaten Informationen über den Hüftgelenkswinkel des Benutzers umfassen, der von einem Winkelsensor der Wearable-Vorrichtung (100) während eines von einem Startpunkt zu einem Endpunkt durchgeführten Gehvorgangs des Benutzers gemessen wird.

12. Verfahren nach Anspruch 11 zum Betreiben des Systems nach Anspruch 2,
wobei das Verfahren ferner Folgendes, das durch die elektronische Vorrichtung (210) durchzuführen ist, umfasst:
Empfangen einer Benutzereingabe für eine Messung einer angestrebten physikalischen Fähigkeit eines Benutzers;
Übertragen des Steuersignals für die Messung der angestrebten physikalischen Fähigkeit als Reaktion auf die Benutzereingabe an die Wearable-Vorrichtung (100);
Empfangen der ersten Sensordaten und der zweiten Sensordaten von der Wearable-Vorrichtung (100);
Bestimmen physikalischer Fähigkeitsinformationen bezüglich der angestrebten physikalischen Fähigkeit des Benutzers auf der Grundlage der ersten Sensordaten und der zweiten Sensordaten,
wobei das Bestimmen Folgendes umfasst:
wenn die elektronische Vorrichtung (210) anhand der empfangenen Sensordaten erkennt, dass der Benutzer das Gehen beendet, Abschließen einer Sammlung von Sensordaten zur Messung einer Gehfähigkeit des Benutzers;
Bestimmen von Informationen über die Gehfähigkeit des Benutzers auf der Grundlage von Sensordaten, die bis zu einem Zeitpunkt erhalten werden, zu dem das Ende des Gehens erkannt wird;
wobei das Bestimmen der Informationen über die Gehfähigkeit das Berechnen eines zeitlichen Gangindex auf der Grundlage der ersten Sensordaten und eines räumlichen Gangindex auf der Grundlage der zweiten Sensordaten sowie das Berechnen der physikalischen Fähigkeitsinformationen auf der Grundlage des zeitlichen Gangindex und des räumlichen Gangindex umfasst; und
Bereitstellen der physikalischen Fähigkeitsinformationen für den Benutzer über einen Benutzeroberflächenbildschirm.

## Revendications

1. Dispositif portable (100), comprenant :
un cadre de support de jambe (50, 55) configuré pour supporter une jambe d'un utilisateur lorsque le dispositif portable (100) est porté sur un corps de l'utilisateur ;
un premier cadre de support de taille (25) et un second cadre de support de taille (20) configurés pour supporter une taille de l'utilisateur lorsque le dispositif portable (100) est porté sur le corps de l'utilisateur ;
un module de capteurs (520), comprenant au moins deux capteurs, configuré pour obtenir des données de capteur comprenant des informations de mouvement de l'utilisateur dans un mode de mesure de capacité physique ;
un module de communication (516), comprenant une circuiterie de communication, configuré pour transmettre les données de capteur à un dispositif électronique (210) et recevoir au moins un signal de commande provenant du dispositif électronique (210) ; et
au moins un processeur (512) configuré pour commander le module de communication (516) et le module de capteurs (520),
dans lequel le module de capteurs (520) est configuré pour obtenir des premières données de capteur comprenant des informations de mouvement de l'utilisateur devant être mesurées par un capteur inertiel et des deuxièmes données de capteur comprenant un angle d'articulation de hanche de l'utilisateur devant être mesuré par un capteur d'angle,
dans lequel les deuxièmes données de capteur comprennent des informations sur l'angle d'articulation de hanche de l'utilisateur mesuré par un capteur d'angle du dispositif portable (100) pendant un processus de marche de l'utilisateur effectué depuis un point de départ jusqu'à un point d'arrivée, et
dans lequel l'au moins un processeur (512) est configuré pour commander le module de communication (516) afin de transmettre les premières données de capteur et les deuxièmes données de capteur au dispositif électronique (210) dans le mode de mesure de capacité physique, dans lequel l'au moins un processeur (512) est en outre configuré pour, lorsque le signal de commande, pour une mesure d'une capacité physique cible de l'utilisateur, est reçu en provenance du dispositif électronique (210) :
activer le mode de mesure de capacité physique en réponse au signal de commande ; et
commander le module de capteurs (520) pour obtenir les données de capteur dans le mode de mesure de capacité physique, et dans lequel l'au moins un processeur (512) est configuré, lorsque le mode de mesure de capacité physique est activé, pour initialiser les données de capteur et commander un enregistrement des données de capteur pour qu'il soit démarré lorsqu'une période de temps prédéterminée pour mesurer la capacité physique cible s'est écoulée.

2. Système comprenant le dispositif portable (100) selon la revendication 1 et un dispositif électronique (210), le dispositif électronique (210) comprenant :
un module de communication (730), comprenant une circuiterie de communication, configuré pour recevoir des données de capteur provenant d'un dispositif portable (100) porté sur une taille et une jambe d'un utilisateur et pour envoyer au moins un signal de commande au dispositif portable (100) ;
au moins un processeur (710) configuré pour déterminer des informations de capacité physique de l'utilisateur sur la base des données de capteur ; et
un module d'affichage (740), comprenant un affichage (212), configuré pour délivrer les informations de capacité physique par l'intermédiaire d'un écran d'interface utilisateur,
dans lequel l'au moins un processeur (710) est configuré pour :
reconnaître que l'utilisateur termine de marcher sur la base des données de capteur reçues, et lorsqu'il est reconnu que l'utilisateur termine de marcher, achever une collecte de données de capteur pour mesurer une capacité de marche de l'utilisateur ; et
déterminer des informations sur la capacité de marche de l'utilisateur sur la base de données de capteur obtenues jusqu'à un instant auquel une fin de la marche est reconnue,
dans lequel l'au moins un processeur est en outre configuré pour :
calculer un indice temporel de marche sur la base de premières données de capteur comprenant des informations de mouvement de l'utilisateur reçues en provenance du dispositif portable (100) ;
calculer un indice spatial de marche sur la base de deuxièmes données de capteur concernant un angle d'articulation de hanche de l'utilisateur reçues en provenance du dispositif portable (100) ; et
calculer les informations de capacité physique sur la base de l'indice temporel de marche et de l'indice spatial de marche,
dans lequel les deuxièmes données de capteur comprennent des informations sur l'angle d'articulation de hanche de l'utilisateur mesuré par un capteur d'angle du dispositif portable (100) pendant un processus de marche de l'utilisateur effectué depuis un point de départ jusqu'à un point d'arrivée.

3. Système selon la revendication 2, dans lequel l'au moins un processeur (710) du dispositif électronique (210) est configuré pour calculer l'indice temporel de marche sur la base d'un intervalle de temps entre des premiers points caractéristiques (1232) temporellement adjacents l'un à l'autre,
d'un intervalle de temps entre des deuxièmes points caractéristiques (1234) temporellement adjacents l'un à l'autre,
et d'un intervalle de temps entre un premier point caractéristique (1232) et un deuxième point caractéristique (1234) temporellement adjacent au premier point caractéristique (1232),
dans lequel l'indice temporel de marche comprend au moins l'un parmi : un temps de pas, un temps d'oscillation, un temps d'appui, un temps d'enjambée ou un temps de double appui de l'utilisateur.

4. Système selon l'une des revendications 2 à 3, dans lequel l'au moins un processeur (710) du dispositif électronique (210) est configuré pour calculer l'indice spatial de marche sur la base d'une valeur d'angle d'articulation de hanche à un instant correspondant à une attaque du talon à partir de la valeur d'angle d'articulation de hanche de l'utilisateur mesurée par le capteur d'angle du dispositif portable (100),
dans lequel l'indice spatial de marche comprend au moins l'un parmi : une longueur de pas, une longueur d'enjambée ou une longueur de jambe de l'utilisateur.

5. Système selon l'une des revendications 2 à 4, dans lequel l'au moins un processeur (710) du dispositif électronique (210) est configuré pour calculer au moins l'un parmi une vitesse de marche, un indice de fluctuation de marche ou un indice de symétrie de marche de l'utilisateur, sur la base de l'indice temporel de marche et de l'indice spatial de marche.

6. Système selon l'une des revendications 2 à 5, dans lequel l'au moins un processeur (710) du dispositif électronique (210) est configuré pour fournir à l'utilisateur, par l'intermédiaire du module d'affichage (740), une interface utilisateur destinée à guider une mesure d'une capacité physique cible de l'utilisateur.

7. Système selon la revendication 6, dans lequel l'au moins un processeur (710) du dispositif électronique (210) est configuré pour, sur la base de la réception d'une entrée utilisateur pour la mesure de la capacité physique cible de l'utilisateur, commander le module de communication (730) afin de transmettre un signal de commande pour la mesure de la capacité physique cible au dispositif portable (100) en réponse à l'entrée utilisateur.

8. Système selon l'une des revendications 2 à 7, dans lequel l'au moins un processeur (710) du dispositif électronique (210) est configuré pour, lorsqu'un mode de mesure de capacité physique destiné à mesurer une capacité physique cible est un test chronométré « se lever et marcher », TUG,
lorsqu'une valeur d'angle d'articulation de hanche comprise dans les données de capteur est supérieure ou égale à un premier seuil, déterminer que l'utilisateur se lève d'une chaise ;
lorsque la valeur d'angle d'articulation de hanche est inférieure ou égale à un deuxième seuil, déterminer que l'utilisateur s'assoit sur la chaise ;
extraire, en tant que point caractéristique, une valeur d'angle d'articulation de hanche correspondant à une valeur minimale parmi des valeurs d'angle d'articulation de hanche mesurées pendant une période de temps prédéfinie qui s'écoule à partir d'un instant auquel il est déterminé que l'utilisateur s'assoit sur la chaise ; et
estimer les informations de capacité physique de l'utilisateur sur la base d'une période de temps allant d'un début du test TUG jusqu'à ce que la valeur d'angle d'articulation de hanche correspondant à la valeur minimale soit extraite.

9. Système selon l'une des revendications 2 à 7, dans lequel l'au moins un processeur (710) du dispositif électronique (210) est configuré pour, lorsqu'un mode de mesure de capacité physique destiné à mesurer une capacité physique cible est un test consistant à s'asseoir et se lever cinq fois, 5xSTS :
lorsqu'une valeur d'angle d'articulation de hanche comprise dans les données de capteur est supérieure ou égale à un premier seuil, déterminer que l'utilisateur se lève d'une chaise et augmenter un nombre de fois où l'utilisateur se lève de la chaise ;
lorsque la valeur d'angle d'articulation de hanche est inférieure ou égale à un deuxième seuil, déterminer que l'utilisateur s'assoit sur la chaise ;
lorsque le nombre de fois où l'utilisateur se lève de la chaise atteint un nombre de fois de référence, extraire, en tant que point caractéristique, une valeur d'angle d'articulation de hanche correspondant à une valeur maximale apparaissant immédiatement avant que le nombre de fois où l'utilisateur se lève de la chaise atteigne le nombre de fois de référence ; et
calculer des informations de capacité physique de l'utilisateur sur la base d'une période de temps allant d'un début du test 5xSTS jusqu'à ce que la valeur d'angle d'articulation de hanche correspondant à la valeur maximale soit extraite.

10. Système selon l'une des revendications 2 à 7, dans lequel l'au moins un processeur (710) du dispositif électronique (210) est configuré pour, lorsqu'un mode de mesure de capacité physique destiné à mesurer une capacité physique cible est un test de batterie courte de performance physique, SPPB :
lorsqu'une valeur d'angle d'articulation de hanche mesurée pendant un intervalle de temps est supérieure ou égale à un premier seuil, déterminer qu'une jambe de l'utilisateur se déplace ; et
calculer les informations de capacité physique de l'utilisateur sur la base d'une posture de l'utilisateur sur laquelle le test SPPB doit être effectué, d'une valeur de mouvement de la partie supérieure du corps mesurée pendant l'intervalle de temps, et d'un instant auquel il est déterminé que la jambe se déplace.

11. Procédé de fonctionnement d'un dispositif portable (100), le dispositif portable (100) comprenant un cadre de support de jambe configuré pour supporter une jambe d'un utilisateur, un premier cadre de support de taille (25) et un second cadre de support de taille (20) configurés pour supporter une taille de l'utilisateur lorsque le dispositif portable (100) est porté sur un corps de l'utilisateur, un module de capteurs (520), comprenant au moins deux capteurs, configuré pour obtenir des données de capteur comprenant des informations de mouvement de l'utilisateur dans un mode de mesure de capacité physique, et un module de communication (516), comprenant une circuiterie de communication, configuré pour transmettre les données de capteur à un dispositif électronique (210) et recevoir au moins un signal de commande provenant du dispositif électronique (210), le procédé comprenant :
la réception d'un signal de commande pour une mesure d'une capacité physique cible de l'utilisateur provenant d'un dispositif électronique (210) ;
l'activation d'un mode de mesure de capacité physique en réponse au signal de commande, lorsque le mode de mesure de capacité physique est activé, l'initialisation de données de capteur et la commande d'un enregistrement des données de capteur pour qu'il soit démarré lorsqu'une période de temps prédéterminée pour mesurer la capacité physique cible s'est écoulée, et l'obtention de premières données de capteur comprenant des informations de mouvement de l'utilisateur mesurées par un capteur inertiel du dispositif portable (100) et de deuxièmes données de capteur comprenant un angle d'articulation de hanche de l'utilisateur mesuré par un capteur d'angle du dispositif portable (100) ; et
la transmission des premières données de capteur et des deuxièmes données de capteur au dispositif électronique (210), de sorte que le dispositif électronique (210) calcule un indice temporel de marche sur la base des premières données de capteur, et calcule un indice spatial de marche sur la base des deuxièmes données de capteur,
dans lequel les deuxièmes données de capteur comprennent des informations sur l'angle d'articulation de hanche de l'utilisateur mesuré par un capteur d'angle du dispositif portable (100) pendant un processus de marche de l'utilisateur effectué depuis un point de départ jusqu'à un point d'arrivée.

12. Procédé, selon la revendication 11, de fonctionnement du système selon la revendication 2,
dans lequel le procédé comprend en outre, par le dispositif électronique (210) :
la réception d'une entrée utilisateur pour une mesure d'une capacité physique cible d'un utilisateur ;
la transmission du signal de commande pour la mesure de la capacité physique cible au dispositif portable (100) en réponse à l'entrée utilisateur ;
la réception des premières données de capteur et des deuxièmes données de capteur provenant du dispositif portable (100) ;
la détermination d'informations de capacité physique concernant la capacité physique cible de l'utilisateur sur la base des premières données de capteur et des deuxièmes données de capteur,
dans lequel la détermination comprend :
lorsque le dispositif électronique (210) reconnaît que l'utilisateur termine de marcher sur la base des données de capteur reçues, l'achèvement d'une collecte de données de capteur pour mesurer une capacité de marche de l'utilisateur ;
la détermination d'informations sur la capacité de marche de l'utilisateur sur la base de données de capteur obtenues jusqu'à un instant auquel la fin de la marche est reconnue ;
dans lequel la détermination des informations sur la capacité de marche comprend le calcul d'un indice temporel de marche sur la base des premières données de capteur et d'un indice spatial de marche sur la base des deuxièmes données de capteur, et le calcul des informations de capacité physique sur la base de l'indice temporel de marche et de l'indice spatial de marche ; et
la fourniture, par l'intermédiaire d'un écran d'interface utilisateur, des informations de capacité physique à l'utilisateur.
